# FASCICULE DE BREVET EUROPEEN

(11) **EP 3 160 425 B1**
(45) Date de publication et mention de la délivrance du brevet: **14.10.2020**
(21) Numéro de dépôt: 15745531.2
(22) Date de dépôt: 30.06.2015
(51) Int. Cl.: A61K 8/25, A61K 8/44, A61Q 1/00, A61K 8/73, A61K 8/37

(54) **COMPOSITION ANHYDRE COMPRENANT UN GELIFIANT LIPOPHILE, AU MOINS DEUX CHARGES DISTINCTES ET UNE PHASE GRASSE**
WASSERFREIE ZUSAMMENSETZUNG ENTHALTEND EIN LIPOPHILES GELIERUNGSMITTEL, MINDESTENS ZWEI VERSCHIEDENEN LADUNGEN UND EINE FETTPHASE
ANHYDROUS COMPOSITION COMPRISING A LIPOPHILIC GELLING AGENT, AT LEAST TWO DISTINCT FILLERS AND A FATTY PHASE

(30) Priorité: 30.06.2014 FR 1456139
(43) Date de publication de la demande: 03.05.2017
(73) Titulaire: L'OREAL, 75008 Paris (FR)
(72) Inventeur: PAGE, Valérie, F-26400 Saoû (FR); GUILBAUD, Sophie, F-26270 Cliousclat (FR); DEMARCQ, Céline, F-26400 Gigors et Lozeron (FR)
(74) Mandataire: L'Oreal
(86) Numéro de dépôt international: PCT/FR2015/051800
(87) Numéro de publication internationale: WO 2016/001578

(56) Documents cités:
- FR-A1- 2 843 018
- FR-A1- 2 975 297

## Description

La présente invention concerne une composition anhydre comprenant de 3 % à 15 % en poids d'au moins un gélifiant lipophile, de 10 à 50 % en poids de charges dont au moins 5 % en poids d'une première charge et au moins 5 % en poids d'une deuxième charge distincte de la première, et de 40 % à 85 % en poids d'au moins une phase grasse. L'invention concerne en particulier le domaine cosmétique, et en particulier le domaine du soin et/ou du maquillage des matières kératiniques, notamment de la peau, des lèvres, des cheveux ou des ongles, de préférence la peau.

Dans le domaine des compositions cosmétiques de soin et/ou de maquillage de la peau, il est connu d'utiliser des charges minérales ou organiques à effet flouteur « soft-focus » qui absorbe le gras, assure un effet optique bluffant « photoshop », ainsi qu'un toucher tout doux, pour matifier la peau, et/ou lisser optiquement le microrelief, combler les rides, camoufler les imperfections de la peau et mieux réfléchir la lumière.

Toutefois, l'utilisation de ces charges s'accompagne en général d'un toucher sec, rèche, de peluches, de traces blanches et d'un manque de confort rhédibitoire pour l'utilisatrice.

Afin de remédier à ce problème et disposer d'un soin confortable ayant un effet flouteur, des galéniques huileuses sont actuellement proposées sur le marché cosmétique. Cependant, elles présentent les inconvénients liés à la présence de forts taux de phase grasse dans la composition, à savoir l'obtention d'une peau luisante et/ou la sensation de peau grasse et/ou collante.

On connaît aussi pour répondre à cette problématique l'utilisation de silicones réticulées. Ce type de matières premières permet en effet de combiner effet mat et soft-focus, mais présente l'inconvénient d'être caractérisé par un toucher gras et chaud peu confortable, avec un effet « masque ».

Ainsi, il est difficile de concilier dans une même composition des performances techniques qui s'opposent telles que par exemple matité (qui peut rendre la peau sèche) et hydratation (qui peut rendre la peau brillante).

Il demeure ainsi difficile pour l'homme de l'art de proposer des compositions homogènes aptes à procurer un résultat visuel immédiat sur la peau avec une sensation de légèreté et de confort à l'application, ce résultat immédiat attendu étant préférentiellement une bonne couvrance des imperfections colorielles et/ou des imperfections de reliefs.

Il subsiste donc le besoin de préparer des compositions cosmétiques résolvant le problème technique de matifier et/ou lisser optiquement le microrelief de la peau, de combler les rides, camoufler les imperfections de la peau et mieux réfléchir la lumière tout en procurant un toucher agréable notamment lors de son application, un fini peau tout doux sans effet « masque » et qui laisse la peau respirer.

La demanderesse a découvert que ce besoin pouvait être satisfait en associant, dans une composition anhydre comprenant une phase grasse, un gélifiant lipophile et au moins deux charges distinctes convenablement sélectionnées, dans des quantités appropriées.

Cette association permet de répondre à la problématique citée ci-dessus en proposant une galénique huileuse, forte d'une sensorialité nouvelle tant à l'application que sur le fini peau sans négliger les qualités cosmétiques.

Plus précisément, la présente invention a pour objet une composition anhydre comprenant :
- de 3 % à 15 % en poids d'au moins un gélifiant lipophile ;
- de 10 à 50 % en poids de charges dont au moins 5 % en poids d'une première charge et au moins 5 % en poids d'une deuxième charge distincte de la première ; et
- de 40 % à 85 % en poids d'au moins une phase grasse ;
les quantités en poids étant données par rapport au poids total de la composition.

La présente invention permet d'obtenir une nouvelle galénique tout particulièrement intéressante au regard de ses performances techniques et des ressentis sensoriels qu'elle procure à l'utilisateur lors de son application sur les matières kératiniques, et en particulier sur la peau.

Ainsi, la composition conforme à l'invention permet de disposer d'un soin confortable anhydre à effet flouteur « soft-focus », c'est-à-dire permettant de matifier et/ou lisser optiquement le microrelief de la peau, combler les rides, camoufler les imperfections de la peau, mieux réfléchir la lumière tout en procurant un toucher agréable et une sensorialité nouvelle, celle d'un soin huileux non gras et non collant, à transformation poudrée notamment lors de son application, avec un fini peau velouté, et qui laisse la peau respirer. La composition répond au besoin des consommatrices d'avoir à disposition un produit huileux sans en avoir les inconvénients d'application et de fini peau engendrés par un type de galénique huileuse classique.

Le confort à l'application se traduit notamment par une absence de tiraillements, de sensations de dessèchement et/ou de sensations collantes et/ou grasses.

La composition selon l'invention est stable, notamment dans le temps et/ou aux variations de température, tout en présentant de bonnes propriétés cosmétiques et sensorielles.

Par stable, on entend stable à température ambiante (25 °C) au moins 1 mois, de préférence au moins 2 mois.

Elle apporte tous les bénéfices d'une huile sans en avoir le toucher gras ni l'aspect luisant. Le fini peau est homogène et confortable. La composition respecte la peau, elle est agréable, ne laisse la peau ni grasse, ni collante. La texture est douce et agréable, la prise facile. Elle apporte un effet optique et tactile pour corriger en surface et immédiatement l'aspect de la peau, en atténuant les zones d'ombre du relief cutané tels que les rides, les ridules, les pores dilatés et autres imperfections cutanées. Elle estompe les signes de fatigue. Elle lisse et affine le grain de la peau, lui redonne éclat et fraicheur, pour un résultat naturel. La présente invention a également pour objet un procédé cosmétique de maquillage et/ou de soin des matières kératiniques comprenant une étape d'application d'une composition telle que définie plus haut sur lesdites matières kératiniques.

La composition de l'invention étant destinée à une application topique sur la peau ou les phanères, elle comprend un milieu physiologiquement acceptable, c'est-à-dire un milieu compatible avec toutes les matières kératiniques telles que la peau, les ongles, les muqueuses et les fibres kératiniques (telles que les cheveux, les cils).

Par « anhydre », au sens de la présente invention, on entend une composition comprenant une teneur inférieure ou égale à 1 % en poids d'eau, de préférence inférieure ou égale à 0,5 % en poids par rapport au poids total de ladite composition, voire exempte d'eau. Le cas échéant, d'aussi faibles quantités d'eau peuvent notamment être amenées par des ingrédients de la composition qui peuvent en contenir des quantités résiduelles.

Dans ce qui va suivre, l'expression « au moins un(e) » est équivalente à « un(e) ou plusieurs » et, à moins d'une autre indication, les bornes d'un domaine de valeurs sont comprises dans ce domaine.

### Gélifiants lipophiles

On entend par « gélifiant lipophile », au sens de la présente invention, un composé apte à gélifier la phase grasse de la composition selon l'invention.

Le ou les gélifiants lipophiles sont liposolubles ou lipodispersibles.

Selon un mode de réalisation particulier de l'invention, le ou les gélifiants lipophiles sont organiques ou minéraux.

Selon un autre mode de réalisation particulier de l'invention, le ou les gélifiants lipophiles sont non siliconés.

Le ou les gélifiants utilisables dans le cadre de l'invention peuvent être des gélifiants lipophiles organiques, polymériques ou moléculaires.

A titre d'exemples de gélifiants lipophiles pouvant être utilisés dans le cadre de l'invention, on peut citer les micas naturels modifiés tel que le fluorosilicate d'aluminium, de magnésium et de potassium, en particulier celui qui est commercialisé par la société SENSIENT sous la dénomination SUBMICA M, les esters de dextrine et d'acide gras tels que le palmitate de dextrine, en particulier celui qui est commercialisé par la société CHIBA FLOUR MILLING sous la dénomination RHEOPEARL TL2-OR, ou le palmitate de dextrine commercialisé par la même société sous la dénomination RHEOPEARL KL2-OR, ainsi que le myristate de dextrine, en particulier celui qui est commercialisé par la société CHIBA FLOUR MILLING sous la dénomination RHEOPEARL MKL2, les tri-esters d'acide gras en C8-C30 et de mono ou polyglycérol tels que le tri-hydroxy-stéarate de glycéryle, en particulier celui qui est commercialisé par la société ELEMENTIS sous la dénomination THIXCIN R ou RHEOCIN commercialisé par la société BYK ADDITIVES & INSTRUMENTS.

Selon un mode de réalisation particulier de l'invention, le ou les gélifiants lipophiles sont organiques.

Selon un mode de réalisation particulier, le ou les gélifiants lipophiles utilisables dans le cadre de l'invention sont choisis parmi les esters de dextrine et d'acide gras et les tri-esters d'acide gras en C8-C30 et de mono ou polyglycérol.

### Esters de dextrine et d'acide gras

Les esters de dextrine et d'acide gras utilisés selon l'invention peuvent être notamment choisis parmi les mono- ou poly-ester de dextrine et d'au moins un acide gras répondant à la formule (C) : 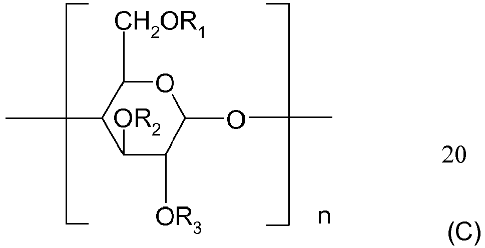 dans laquelle :
n est un entier allant de 3 à 150, notamment de 10 à 100, et de préférence de 15 à 40 ;
les radicaux R₁, R₂ et R₃ représentent un atome d'hydrogène ou un groupement acyle (R-CO-) dans lequel le radical R est un groupement hydrocarboné, linéaire ou ramifié, saturé ou insaturé, possédant 6 à 50, notamment 8 à 30, voire 12 à 22, et mieux 12 à 18 atomes de carbone, sous réserve qu'au moins un desdits radicaux R₁, R₂ et R₃ est différent d'un atome d'hydrogène.

En particulier, R₁, R₂ et R₃ peuvent représenter l'hydrogène ou un groupement acyle (R-CO-) dans lequel R est un radical hydrocarboné tel que défini précédemment, sous réserve qu'au moins deux desdits radicaux R₁, R₂ ou R₃ sont identiques et différents de l'hydrogène. L'ensemble des radicaux R₁, R₂ et R₃ peuvent figurer un groupement acyle (R-CO) identique ou différent et notamment identique.

Le radical R-CO- de l'ester de dextrine de formule (C) peut notamment être choisi parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

Le radical R-CO est avantageusement linéaire. R-CO est de préférence le radical palmityle ou le radical myristyle, et encore plus préférentiellement le radical palmityle.

n va avantageusement de 25 à 35, de préférence va de 27 à 33, et mieux est égal à 30.

De préférence, on utilise à titre d'ester de dextrine et d'acide(s) gras au moins un palmitate de dextrine et/ou au moins un myristate de dextrine. Ceux-ci peuvent être utilisé seuls ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine sont disponibles commercialement, en particulier des palmitates de dextrine, par exemple sous la dénomination RHEOPEARL TL2-OR ou RHEOPEARL KL2-OR de la société CHIBA FLOUR MILLING, et sous la dénomination RHEOPEARL KS de la société CHIBA FLOUR MILLING, et des myristates de dextrine, par exemple sous la dénomination RHEOPEARL MKL2 de la société CHIBA FLOUR MILLING.

Selon un mode de réalisation particulier de l'invention, on utilisera un mélange d'un ester de dextrine et d'acide gras dont le degré de substitution est inférieur à 2 sur la base d'une unité glucose et d'un ester de dextrine et d'acide gras dont le degré de substitution est supérieur à 2 sur la base d'une unité glucose, comme décrit dans la demande FR 2 843 020.

Selon un mode de mise en oeuvre, l'ester de dextrine et d'acide gras dont le degré de substitution est inférieur à 2 sur la base d'une unité glucose répond avantageusement à la formule (IV) suivante : 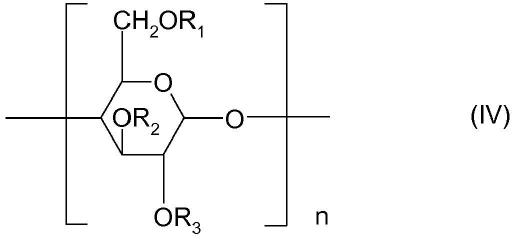 dans laquelle :
les radicaux R₁, R₂ et R₃ représentent un atome d'hydrogène ou un groupement acyle (R-CO-) dans lequel le radical R est un groupement hydrocarboné, linéaire ou ramifié, saturé ou insaturé, possédant 6 à 50, notamment 8 à 30, voire 12 à 22 et mieux 12 à 18 atomes de carbone, sous réserve qu'au moins un desdits radicaux R₁, R₂ et R₃ est différent d'un atome d'hydrogène ;
n est un entier allant de 3 à 150, notamment de 10 à 100, et de préférence de 15 à 40.

Le radical R-CO- de l'ester de dextrine de formule (IV) peut notamment être choisi parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitoléyle, oléyle, élaidyle, eicosényle, sorbyle, linoléyle, linolényle, punicyle, arachidonyle, stéarolyle, et leurs mélanges.

Le radical R-CO est avantageusement linéaire. Le radical R-CO est de préférence le radical palmityle ou le radical myristyle, et encore plus préférentiellement le radical palmityle.

n va avantageusement de 25 à 35, de préférence va de 27 à 33, et mieux est égal à 30.

De préférence, on utilise un ester de dextrine d'acide gras dont le degré de substitution est inférieur à 2 sur la base d'une unité glucose, tel que le degré de substitution est inférieur à 1,9, de préférence inférieur à 1,8, de préférence encore est compris entre 1,5 et 1,7. Certains de ces esters de dextrine sont disponibles commercialement, notamment sous la dénomination RHEOPEARL TL de la société Chiba Flour Milling.

Le poids moléculaire moyen en poids de l'ester de dextrine et d'acide gras dont le degré de substitution est inférieur à 2 sur la base d'une unité glucose est de préférence compris entre 10.000 et 30.000, de préférence encore entre 15.000 et 20.000. Le poids moléculaire moyen en poids est déterminé en chromatographie en phase gazeuse, avec un étalonnage polystyrène.

Selon un mode de mise en oeuvre, l'ester de dextrine et d'acide gras dont le degré de substitution est supérieur à 2 sur la base d'une unité glucose répond à la formule (V) : 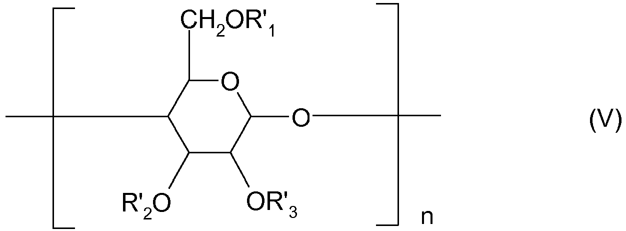 dans laquelle :
les radicaux R'₁, R'₂ et R'₃, identiques ou différents, sont choisis parmi un atome d'hydrogène ou un groupement acyle (R'-CO-) dans lequel le radical R' est un groupement hydrocarboné, linéaire ou ramifié, saturé ou insaturé, possédant 6 à 50, notamment 8 à 30, voire 12 à 22 et mieux 12 à 18 atomes de carbone, sous réserve qu'au moins un desdits radicaux R'₁, R'₂ et R'₃ est différent d'un atome d'hydrogène ;
n est un entier allant de 3 à 150, notamment de 10 à 100, et de préférence de 15 à 40.
R' et n peuvent avoir la même signification que R et n décrits précédemment.

Avantageusement, les radicaux R'₁, R'₂ et R'₃ sont identiques, sous réserve qu'au moins un desdits radicaux R'₁, R'₂ et R'₃ est différent d'un atome d'hydrogène.

De préférence, on utilise un ester de dextrine d'acide gras dont le degré de substitution est supérieur à 2 sur la base d'une unité glucose, tel que le degré de substitution est supérieur à 2,1, de préférence compris entre 2,1 et 2,3.

Le poids moléculaire moyen en poids de l'ester de dextrine et d'acide gras dont le degré de substitution est supérieur à 2 sur la base d'une unité glucose est de préférence compris entre 10.000 et 30.000, de préférence encore entre 15.000 et 20.000. Le poids moléculaire moyen en poids est déterminé en chromatographie en phase gazeuse, avec un étalonnage polystyrène.

A titre d'exemples d'esters de dextrine de formule (V) selon l'invention, on peut citer le Rheopearl KL vendu par la société CHIBA FLOUR MILLING.

### Tri-esters d'acide gras et de mono ou polyglycérol

Selon un mode de réalisation particulier, le ou les tri-esters d'acide gras et de mono ou polyglycérol sont des tri-esters d'acide gras et de monoglycérol.

Par acide gras, on entend un acide linéaire ou ramifié, saturé ou insaturé, comprenant de 8 à 30 atomes de carbone, de préférence de 10 à 24 atomes de carbone, encore plus préférentiellement de 12 à 22, et mieux de 16 à 20 atomes de carbone, substitué ou non substitué par un ou plusieurs groupements hydroxyle. Selon un mode de réalisation particulier de l'invention, le ou les acides gras sont linéaires, saturés, substitués par au moins un groupement hydroxyle.

Les acides gras peuvent être choisis parmi l'acide oléique, l'acide laurique, l'acide palmitique, l'acide myristique, l'acide stéarique, l'acide linoléique, l'acide caprique, l'acide béhénique, substitués ou non par au moins un groupement hydroxyle, ou leurs mélanges. De préférence, le ou les acides gras sont choisis parmi l'acide stéarique, les acides stéariques substitués par au moins un groupement hydroxyle, et leurs mélanges, plus préférentiellement ils sont choisis parmi l'acide stéarique, l'acide 12-hydroxy stéarique, et leurs mélanges, et encore mieux, il s'agit de l'acide 12-hydroxy stéarique.

Selon un mode de réalisation particulier de l'invention, le tri-ester d'acide gras en C8-C30 et de mono ou polyglycéryle est le tris(12-hydroxystéarate) de glycéryle.

A titre d'exemples de tri-ester d'acide gras en C8-C30 et de mono ou polyglycérol, on peut citer le tri-(hydroxystérate) de glycéryle (nom INCI : TRIHYDROXYSTEARIN) comme par exemple celui qui est commercialisé par la société ELEMENTIS sous la dénomination THIXCIN R ou celui qui est commercialisé par la société BYK ADDITIVES & INSTRUMENTS sous la dénomination RHEOCIN.

Selon un mode de réalisation particulier de l'invention, le ou les gélifiants lipophiles utilisables dans le cadre de l'invention sont choisis parmi les esters de dextrine et d'acide gras, de préférence le palmitate de dextrine et le myristate de dextrine.

Selon un autre mode de réalisation particulier de l'invention, le ou les gélifiants lipophiles utilisables dans le cadre de l'invention sont choisis parmi les tri-esters d'acide gras en C8-C30 et de monoglycérol, de préférence les tri(hydroxystéarate) de glycéryle, et encore plus préférentiellement le tris(12-hydroxystéarate) de glycéryle.

Le ou les gélifiants lipophiles sont présents dans la composition conforme à l'invention en quantité comprise entre 3 % et 15 % en poids du poids total de la composition, de préférence de 5 % à 12 % en poids, et encore plus préférentiellement de 8,5 % à 10 % en poids.

### Charges

La composition conforme à l'invention comprend de 10 à 50 % en poids de charges par rapport au poids total de la composition dont au moins 5 % en poids d'une première charge et au moins 5 % en poids d'une deuxième charge distincte de la première.

Par charge, il faut comprendre des particules de toute forme, incolores ou blanches, minérales ou de synthèse, insolubles dans le milieu de la composition quelle que soit la température à laquelle la composition est fabriquée.

La ou les charges peuvent être notamment des charges organiques et/ou des charges inorganiques.

Les charges utilisées dans la présente invention peuvent être caractérisées par leur surface spécifique par unité de masse ou par unité de volume, leur taille exprimée en diamètre moyen en volume D(4,3), leur densité non tassée, et/ou leur capacité d'absorption d'huile. Les tailles des charges peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La surface spécifique par unité de masse peut être déterminée par la méthode d'absorption d'azote appelée méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.

La surface spécifique par unité de volume est donnée par la relation : S_{V} = S_{M} x ρ ; où ρ est la densité tassée exprimée en g/cm3 et S_{M} est la surface spécifique par unité de masse exprimée en m²/g, telles que définie plus haut.

Dans le cadre de la présente invention, cette densité peut être appréciée selon le protocole suivant, dit de la densité tassée :
On verse 40 g de poudre dans une éprouvette graduée; puis on place l'éprouvette sur l'appareil STAV 2003 de chez STAMPF VOLUMETER ; l'éprouvette est ensuite soumise à une série de 2500 tassements (cette opération est recommencée jusqu'à ce que la différence de volume entre 2 essais consécutifs soit inférieure à 2%); puis on mesure directement sur l'éprouvette le volume final Vf de poudre tassée. La densité tassée est déterminée par le rapport m/Vf, en l'occurrence 40/Vf (Vf étant exprimé en cm³ et m en g).

La capacité d'absorption d'huile mesurée au Wet Point, et notée Wp, correspond à la quantité d'huile qu'il faut additionner à 100 g de particules pour obtenir une pâte homogène.

Elle est mesurée selon la méthode dite de Wet Point ou méthode de détermination de prise d'huile de poudre décrite dans la norme NF T 30-022. Elle correspond à la quantité d'huile adsorbée sur la surface disponible de la poudre et/ou absorbée par la poudre par mesure du Wet Point, décrite ci-dessous :
On place une quantité m= 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.

La prise d'huile correspond au rapport Vs / m.

Selon un mode de réalisation particulier, les charges utilisées dans la présente invention une capacité d'absorption d'huile de 0,25 mL/g à 3,5 mL/g, de préférence de 0,93 mL/g à 2,5 mL/g, voire de 1,25 mL/g à 2,5 mL/g.

Selon un mode de réalisation particulier, les charges utilisées dans la présente invention présentent une taille exprimée en diamètre moyen en volume D(4,3) allant de 0,1 µm à 40 µm, de préférence de 0,5 µm à 20 µm, et encore plus préférentiellement de 1 µm à 16 µm. Selon un mode de réalisation particulier de l'invention, les charges utilisées dans la présente invention ont une granulométrie hétérogène, c'est-à-dire une grande répartition de taille pour une taille exprimée en diamètre moyen en volume donnée.

Selon un mode de réalisation particulier, les charges utilisées dans la présente invention présentent une densité non tassée allant de 0,2 g/cm³ à 2,2 g/cm³.

### Charges organiques

On entend dans la présente demande par « charge organique », tout solide organique insoluble dans le milieu à température ambiante (25 °C).

On entend par « organique », tout composé ou polymère dont la structure chimique comprend au moins un ou plusieurs atomes de carbone.

Les charges organiques sont choisies parmi les particules sphériques de cellulose, les poudres d'acide aminé N-acylé ayant un groupe acyle en C8-C22, et les particules de polyamide.

On entend dans le cadre de la présente invention par « particules sphériques » des particules ayant la forme ou sensiblement la forme d'une sphère, insolubles dans le milieu de la composition selon l'invention, même à la température de fusion du milieu (environ 100 °C). Selon un mode de réalisation particulier, les particules sphériques de cellulose utilisables dans le cadre de l'invention sont des microparticules. De préférence, elles ont une taille exprimée en diamètre moyen en volume D(4,3) de particule allant de 0,1 à 35 µm, de préférence de 1 à 20 µm, et plus particulièrement de 4 à 15 µm.

A titre d'exemples de microparticules sphériques de cellulose, on peut notamment citer les billes solides de cellulose commercialisées sous les dénominations CELLULOBEADS D-10, CELLULOBEADS D-5 et CELLULOBEADS USF par la société DAITO KASEI KOGYO.

Les acides aminés N-acylés comprennent un groupe acyle ayant de 8 à 22 atomes de carbones, comme par exemple un groupe 2-éthyl hexanoyle, caproyle, lauroyle, myristoyle, palmitoyle, stéaroyle, cocoyle. L'acide aminé peut être par exemple la lysine, l'acide glutamique, l'alanine, de préférence la lysine.

Selon un mode de réalisation particulier, le ou les acides aminés N-acylés comprennent un groupe acyle ayant de 10 à 14 atomes de carbone. De préférence, il s'agit du groupe lauroyle. Avantageusement, la poudre d'acide aminé N-acylé peut être une poudre de lauroyl lysine telle que celle qui est commercialisée sous la dénomination AMIHOPE LL par la société AJINOMOTO ou encore celle qui est commercialisée sous la dénomination CORUM 5105 S par la société CORUM.

### Charges inorganiques

On entend dans la présente demande par « charge inorganique », tout solide inorganique insoluble dans le milieu à température ambiante (25 °C).

On entend par « inorganique », tout composé ou polymère dont la structure chimique ne comprend pas d'atome de carbone.

Il s'agit des particules de silice sphériques poreuses ayant une taille exprimée en diamètre moyen en volume D(4,3) de particule allant de 0,5 à 30 µm, plus particulièrement de 1 à 20 µm, et préférentiellement de 1 à 16 µm.

On entend dans la présente demande par « particules sphériques » des particules ayant la forme ou sensiblement la forme d'une sphère, insolubles dans le milieu de la composition selon l'invention, même à la température de fusion du milieu (environ 100 °C).

Selon un mode de réalisation particulier, elles ont une surface spécifique allant de 30 à 1000 m²/g, et plus particulièrement de 150 à 800 m²/g.

Selon un autre mode de réalisation particulier, elles ont une capacité d'absorption en huile allant de 0,15 à 5 ml/g et plus particulièrement de 1,30 à 1,90 ml/g.

A titre d'exemple de microbilles de silice poreuse, on peut utiliser les produits commerciaux suivants : Silica Beads SB-150, SB-300 ou encore SB 700, préférentiellement SB 300 de la société MYOSHI KASEI ; la gamme des SUNSPHERE de la société Asahi Glass AGC SI-TECH notamment la Sunsphere H-51 ou encore les Sunsphère 12L, Sunsphère H-201, H-52 et H-53 ; Sunsil 130 de la société Sunjin ; Spherica P-1500 de la société Ikeda Corporation ; Sylosphere de la société Fuji Silysia ; les gammes des Silica Pearl et Satinier de la société JGC Catalysts and Chemicals, plus particulièrement la Satinier M13 et M16, les silices MSS-500 de la société KOBO, et plus particulièrement la MSS-500-20N, ainsi que la Silica Shells de la société KOBO.

La composition comprend au moins deux charges distinctes les unes des autres dont l'une est choisie parmi les particules sphériques de silice poreuse, en particulier les microparticules sphériques de silice poreuse, les poudres d'acide aminé N-acylé ayant un groupe acyle en C8-C22 et les particules de polyamide, et l'autre est choisie parmi les particules sphériques de silice poreuse, en particulier les microparticules sphériques de silice poreuse.

La composition conforme à l'invention comprend de 10 % à 50 % en poids, de préférence de 15 % à 35 % en poids du poids total de la composition, de charges.

La composition conforme à l'invention comprend au moins 5 % en poids d'une première charge, de préférence 5 % à 25 % en poids du poids total de la composition, et au moins 5 % en poids d'une deuxième charge, de préférence de 5 % à 25 % en poids du poids total de la composition.

### Phase grasse

La composition conforme à l'invention comprend de 40 % à 85 % en poids du poids total de la composition d'une phase grasse.

Selon un mode de réalisation particulier, la composition conforme à l'invention comprend de 45 % à 85 % en poids d'une phase grasse. De préférence, la proportion de la phase grasse va de 50 % à 85 % en poids, et encore plus préférentiellement de 54 % à 77 % en poids du poids total de la composition.

Cette quantité indiquée ne comprend pas la teneur en gélifiants lipophiles tels que ceux qui sont décrits ci-dessus.

Au sens de l'invention, la phase grasse inclut tout corps gras liquide à température ambiante et pression atmosphérique, généralement des huiles, ou solide à température ambiante et pression atmosphérique, à l'image des cires, ou tout composé pâteux, présents dans ladite composition.

Le ou les corps gras présents dans la composition peuvent être choisis par l'homme du métier sur la base de ses connaissances générales, de manière à conférer à la composition finale les propriétés souhaitées, notamment en termes de propriétés rhéologiques (mesure de pénétrométrie, écoulement, consistance), en termes de texture et de stabilité.

### Composé pâteux

Par "composé pâteux" au sens de la présente invention, on entend un composé gras lipophile à changement d'état solide/liquide réversible et comportant à la température de 23 °C une fraction liquide et une fraction solide.

Un composé pâteux est à la température de 23 °C, sous la forme d'une fraction liquide et d'une fraction solide. En d'autres termes, la température de fusion commençante du composé pâteux est inférieure à 23 °C. La fraction liquide du composé pâteux, mesurée à 23 °C, représente de 20 à 97 % en poids du composé pâteux. Cette fraction liquide à 23 °C représente plus préférentiellement de 25 à 85 %, et mieux de 30 à 60 % en poids du composé pâteux.

La fraction liquide en poids du composé pâteux à 23 °C est égale au rapport de l'enthalpie de fusion consommée à 23 °C sur l'enthalpie de fusion du composé pâteux.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

La fraction liquide du composé pâteux, mesurée à 32 °C, représente de préférence de 40 à 100 % en poids du composé pâteux, mieux encore de 50 à 100 % en poids du composé pâteux. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux, mesurée à 32 °C, est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Le composé pâteux a de préférence une dureté à 20 °C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20 °C au centre de 5 échantillons. Le cylindre est introduit dans chaque échantillon, la profondeur de pénétration étant de 0,3 mm. La valeur relevée de la dureté est celle du pic maximum.

Le composé pâteux est choisi parmi les composés d'origine végétale. Un composé pâteux peut être obtenu par synthèse à partir de produits de départ d'origine végétale.

Le composé pâteux peut être choisi notamment parmi l'huile de jojoba isomérisée telle que l'huile de jojoba partiellement hydrogénée isomérisée trans fabriquée ou commercialisée par la société Desert Whale sous la référence commerciale Iso-Jojoba-50®, la cire d'orange comme, par exemple, celle qui est commercalisée sous la référence Orange Peel Wax par la société Koster Keunen, le beurre de cupuacu (Rain forest RF3410 de la société Beraca Sabara), le beurre de murumuru (RAIN FOREST RF3710 de la société Beraca Sabara), le beurre de karité, l'huile d'olive partiellement hydrogénée comme, par exemple, le composé commercialisé sous la référence Beurrolive par la société Soliance, le beurre de cacao, l'huile de mangue comme, par exemple, la Lipex 203 de la société Aarhuskarlshamn et leurs mélanges.

On peut également citer les mélanges d'acides gras comprenant de 8 à 30 atomes de carbone et d'alcool gras comprenant de 8 à 30 atomes de carbone tels les mélanges d'alcool laurique et méthyl laurate, d'alcool stéarique et méthyl palmitate ou méthyl béhénate telle la gamme des Purester commercialisée par la société Strahl & Pitsch, notamment le lauryl laurate connu sous la dénomination commerciale de Purester 24.

De même, on pourra citer les esters d'acide gras comprenant de 8 à 30 atomes de carbone et de polyglycérol comprenant de 2 à 10 unités glycéryles tels que le Polyglyceryl-3 polyricinoleate commercialisé par la société Aarhuskarlshamn sous la dénomination Akoline PGPR ou encore un mélange de 3 cires jojoba esters & helianthus annus seed wax & acacia decurrens extract et de polyglycéryle tel le produit commercialisé sous la dénomination d'Hydracire S ou Acticire® par la société Gatefossé.

On peut également citer les esters de glycérol hydrogénés tels que par exemple le produit commercialisé sous la dénomination Cegesoft HF 52 par la société COGNIS (BASF), mélange d'huiles de colza et de Palme hydrogénées ou encore le produit commercialisé sous la dénomination Softisan 100 Cremer par la société OLEO.

On peut également citer les mélanges de mono et/ou diester d'acide gras comprenant de 8 à 18 atomes de carbone et de mono ou polyglycérol tel que le stéarate de glycéryle comme par exemple le glyceryl stearate commercialisé sous la référence CUTINA GMS V par la société Cognis ou encore le mélange de mono et distearate de glycéryle commercialisé par la société STEARINERIE DUBOIS sous la dénomination DUB GMS 50/50,

Lorsqu'ils sont présents, la quantité de composés pâteux peut aller par exemple de 0,05 % à 85 % en poids, mieux de 0,1 à 40 % en poids, en particulier de 0,5 à 10 % en poids par rapport au poids total de la composition.

### Cires

Outre un tel corps gras pâteux, la composition de l'invention peut également comprendre au moins une cire.

Par cire au sens de la présente invention, on entend un composé lipophile, solide à température ambiante (25 °C), à changement d'état solide/liquide réversible, ayant une température de fusion supérieure ou égale à 30 °C, et pouvant aller jusqu'à 120 °C. En particulier, les cires présentent une température de fusion supérieure à 30 °C, et mieux supérieure à 45 °C.

Au sens de l'invention, la température de fusion correspond à la température du pic le plus endothermique observé en analyse thermique (analyse calorimétrique différentielle ou DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :
Un échantillon de 5 mg de cire disposé dans un creuset est soumis à une première montée en température allant de - 20 °C à 100 °C, à la vitesse de chauffe de 10 °C/minute, puis est refroidi de 100 °C à - 20 °C à une vitesse de refroidissement de 10 °C/minute et enfin soumis à une deuxième montée en température allant de - 20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Par cire dure, on entend au sens de la présente invention, une cire présentant à 20 °C, une dureté supérieure à 5 MPa, notamment allant de 5 à 30 MPa, de préférence supérieure à 6 MPa, mieux encore allant de 6 à 25 MPa.

La dureté de la cire est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant : on fait fondre la cire à une température égale à la température de fusion de la cire + 10 °C. On fait couler la cire fondue dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

Comme cire, on peut utiliser avantageusement les cires d'origine végétale telles que la cire d'abeille, notamment celle qui est commercialisée sous la dénomination WHITE BEESWAX SP 453P par la société STRAHL & PITSCH ou encore CERABEIL LOR par la société BAERLOCHER, la cire de blé noir telle que celle qui est commercialisée par la société CODIF, la cire de carnauba, la cire de candellila, notamment la référence commerciale CANDELILLA WAX SP 75 G de la société STRAHL & PITSCH, la cire de jojoba hydrogénée, la cire de sumac, les cires obtenues par hydrogénation d'huile d'olive estérifiée avec les alcool gras à chaîne en C12 à C18 vendues par la société SOPHIM dans la gamme Phytowax (12L44, 14L48, 16L55 et 18L57), la cire de son de riz, les alcools cétylique, stéarylique et béhénique, la cire de laurier, la cire d'Ouricury.

On peut également utiliser au moins un ester d'acide béhénique et de glycérol, et en particulier un mélange d'esters d'acide béhénique et de glycérol comme par exemple le mélange glyceryl dibehenate, tribehenin, glyceryl behenate commercialisé par la société Gattefossé sous la référence COMPRITOL 888 CG ATO.

Lorsqu'elles sont présentes, les cires peuvent être présentes en une teneur allant de 0,01 à 40 % en poids, de préférence de 0,05 à 10 % en poids, et mieux de 0,1 à 5 % en poids par rapport au poids total de la composition.

### Huiles

La composition selon l'invention comprend avantageusement au moins une huile.

Par huiles, on entend les corps gras liquides à température ambiante (25 °C) et pression atmosphérique.

Comme huiles utilisables dans la composition de l'invention, on peut citer par exemple :
- les huiles hydrocarbonées d'origine végétale, telle que le squalane, les triglycérides liquides d'acides gras comportant de 4 à 30 atomes de carbone comme les triglycérides des acides heptanoïque ou octanoïque ou encore, par exemple, les huiles de jojoba, de babassu, de tournesol, d'olive, de noix de coco, de noix du brésil, de marula, de maïs, de soja, de courge, de pépins de raisin, de lin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de beurre de karité ;
- les esters et les éthers de synthèse, notamment d'acides et/ou d'alcools gras, comme les huiles de formules R¹COOR² et R¹OR² dans laquelle R¹CO représente le reste d'un acide gras ou R¹ représente le reste d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- leurs mélanges.

On peut citer également les huiles suivantes :
- les esters issus de la réaction d'au moins un acide gras comportant au moins 6 atomes de carbone, de préférence de 6 à 26 atomes de carbone, et mieux de 6 à 20 atomes de carbone, encore mieux de 6 à 16 atomes de carbone et d'au moins un alcool comprenant de 1 à 17 atomes de carbone et mieux de 3 à 15 atomes de carbone ; on peut citer notamment le myristate d'isopropyle tel que ceux qui sont commercialisés sous la dénomination PALMESTER 1510 par la société KLK OLEO, sous la dénomination LEXOL IPM-NF par la société INOLEX CHEMICAL COMPANY ou encore sous la dénomination ISOPROPYLMYRISTATE par la société COGNIS (BASF), l'isostéarate d'isopropyle tel que celui qui est commercialisé sous la dénomination RADIA 7739 par la société OLEON, le palmitate d'isopropyle, le caprate/caprylate d'ethyl2-hexyle (ou caprate/caprylate d'octyle), le palmitate d'éthyl-2-hexyle, le néopentanoate d'isostéaryle, l'isononanoate d'isononyle, le laurate d'hexyle, les esters d'acide lactique et d'alcools gras comprenant 12 ou 13 atomes de carbone, le carbonate de dicaprylyle tel que celui qui est commercialisé sous la dénomination CETIOL CC par la société COGNIS,
- les éthers d'alcools gras comprenant de 6 à 20 atomes de carbone, de préférence de 8 à 12, et encore plus préférentiellement de 8 à 10.

Ces éthers peuvent être obtenus à partir de deux alcools gras différents ou de deux alcools gras identiques. De préférence, ils sont obtenus à partir de deux alcools gras identiques tel que l'alcool caprylique (encore appelé octan-1-ol ou n-octanol). L'éther correspondant est alors le dicaprylyl éther tel que celui qui est commercialisé sous la dénomination Cetiol OE par la société Cognis.
- les éthers de glycérol comprenant de 6 à 12 atomes de carbone comme le 2-éthyl hexyle éther de glycérol (nom INCI : ethylhexylglycerin) tel que le Sensiva SC 50 de la société Schulke & Mayr GmbH.

On peut en particulier citer le mélange d'esters d'acides caprylique/caprique et d'alcools gras en C12-C18 tel que le coco-caprylate/ caprate commercialisé sous la dénomination CETIOL LC par la société COGNIS ou encore sous la dénomination DUB 810 C par la société STEARINERIE DUBOIS.
- les alcanes linéaires volatils, avantageusement d'origine végétale, comprenant de 7 à 17 atomes de carbone, en particulier de 9 à 15 atomes de carbone, et plus particulièrement de 11 à 13 atomes de carbone.

A titre d'exemples d'alcanes linéaires volatils convenant à l'invention, on peut mentionner ceux décrits dans la demande de brevet de la société Cognis WO 2007/068371.

A titre d'exemple d'alcane linéaire volatil convenant à l'invention, on peut citer le n-nonane (C₉), le n-décane (C₁₀), le n-undécane (C₁₁), le n-dodécane (C₁₂), le n-tridécane (C₁₃), le n-tétradecane (C₁₄), le n-pentadécane (C₁₅), le n-héxadécane (C₁₆) et le n-heptadécane (C₁₇) et leurs mélanges. Selon une forme particulièrement préférée, on utilisera un mélange d'undécane (C₁₁) et de tridécane (C₁₃) comme le produit commercialisé sous la référence de CETIOL UT par la Société Cognis. On peut également citer le n-dodécane (C12) et le n-tétradécane (C14) tels que ceux vendus par Sasol respectivement sous les références PARAFOL 12-97 et PARAFOL 14-97, ainsi que leurs mélanges.
- les polyesters obtenus par condensation de dimère et/ou trimère d'acide gras insaturé en C8-C30 et de diol comme par exemple les polyesters d'acide dilinoléique et de diol commercialisés par Biosynthis sous la dénomination Viscoplast et notamment le polymère portant le nom INCI dilinoleic acid/propanediol copolymer ;
- et leurs mélanges.

On peut également utiliser des alcools de Guerbet ou des dérivés d'alcools de Guerbet tels que par exemple des esters d'alcools de Guerbet et d'acide gras en C8-C30. Les alcools de Guerbet sont obtenus par conversion d'un alcool primaire aliphatique en un dimère d'alcool béta-alkyléné via la réaction chimique suivante :

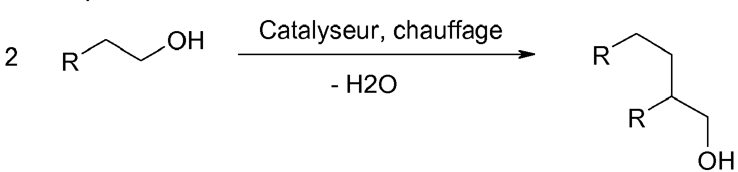

Cette réaction nécessite la présence d'une base comme les hydroxydes de métaux alcalins ou les alcoxydes de métaux alcalins, d'un catalyseur tel que le Nickel de Raney, et de fortes températures.

A titre d'alcools de Guerbet ou d'esters d'acides gras en C8-C30 et d'alcools de Guerbet, on peut notamment citer l'octyldodécanol et les esters d'octyldodécanol tels que le myristate d'octyldodécyle. On peut citer en particulier l'octydodécanol tel que celui qui est commercialisé sous la dénomination EUTANOL G par la société COGNIS (BASF) et le myristate d'octyldodécyle commercialisé sous la dénomination DUB MOD par la société GATTEFOSSE.

La composition selon l'invention peut présenter une teneur totale en huiles allant de 40 % à 85 % en poids, de préférence de 50 % à 85 % en poids, mieux de 54 % à 77 % en poids par rapport au poids total de la composition.

Selon un mode de réalisation particulier de l'invention, la phase grasse de la composition comprend au moins une huile choisie parmi les huiles hydrocarbonées d'origine végétale, de préférence les triglycérides, les alcools de Guerbet, les esters d'acide gras en C8-C30 et d'alcool de Guerbet, les alcanes linéaires en C7-C17, et leurs mélanges.

Selon un mode de réalisation particulier, la composition comprend de 40 % à 85 % en poids d'au moins une huile choisie parmi les huiles hydrocarbonées d'origine végétale, de préférence les triglycérides, les alcools de Guerbet, les esters d'acide gras en C8-C30 et d'alcool de Guerbet, les alcanes linéaires en C7-C17, et leurs mélanges.

Selon un autre mode de réalisation particulier de l'invention, la composition comprend de 15 % à 85 % en poids d'au moins une huile choisie parmi les huiles hydrocarbonées d'origine végétale, de préférence les triglycérides, les alcools de Guerbet et les esters d'acide gras en C8-C30 et d'alcool de Guerbet, et éventuellement de 5 % à 25 % en poids, de préférence de 5 % à 10 % en poids d'au moins une huile choisie parmi les alcanes linéaires en C7-C17.

Selon un premier mode de réalisation spécifique de l'invention, la composition anhydre comprend :
- de 3 % à 15 % en poids d'au moins un gélifiant lipophile choisi parmi les esters de dextrine et d'acide gras, de préférence le palmitate de dextrine ;
- de 10 à 50 % en poids de charges dont au moins 5 % en poids de microparticules sphériques de silice poreuse et au moins 5 % en poids de poudre d'acide aminé N-acylé ayant un groupe acyle en C8-C22, de préférence avec un rapport massique R silice / acide aminé N-acylé ayant un groupe acyle en C8-C22 supérieur ou égal à 0,75, de préférence compris entre 0,75 et 3 ; et
- de 40 % à 85 % en poids d'au moins une phase grasse comprenant au moins une huile choisie parmi les huiles hydrocarbonées d'origine végétale, de préférence les triglycérides, les alcools de Guerbet et les esters d'acide gras en C8-C30 et d'alcool de Guerbet, et les alcanes linéaires en C7-C17 ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine ;
- de 10 à 50 % en poids de charges dont au moins 5 % en poids de microparticules sphériques de silice poreuse et au moins 5 % en poids de poudres d'acide aminé N-acylé ayant un groupe acyle en C8-C22, de préférence avec un rapport massique R silice / acide aminé N-acylé ayant un groupe acyle en C8-C22 supérieur ou égal à 0,75, et encore plus préférentiellement compris entre 0,75 et 3 ; et
- de 40 % à 85 % en poids d'au moins une phase grasse comprenant au moins une huile choisie parmi les huiles hydrocarbonées d'origine végétale, de préférence les triglycérides ; les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 10 à 50 % en poids de charges dont au moins 5 % en poids de microparticules sphériques de silice poreuse et au moins 5 % en poids de poudres d'acide aminé N-acylé ayant un groupe acyle en C8-C22, de préférence avec un rapport massique R silice / acide aminé N-acylé ayant un groupe acyle en C8-C22 supérieur ou égal à 0,75, et encore plus préférentiellement compris entre 0,75 et 3 ; et
- de 40 % à 85 % en poids d'au moins une phase grasse comprenant de 25 % à 48 % en poids d'au moins une huile choisie parmi les huiles hydrocarbonées d'origine végétale, de préférence les triglycérides ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine;
- de 10 % à 50 % en poids de charges dont au moins 5 % en poids de microparticules sphériques de silice poreuse et au moins 5 % en poids de poudres d'acide aminé N-acylé ayant un groupe acyle en C8-C22, de préférence avec un rapport massique R silice / acide aminé N-acylé ayant un groupe acyle en C8-C22 supérieur ou égal à 0,75, et encore plus préférentiellement compris entre 0,75 et 3 ; et
- de 40 % à 85 % en poids d'au moins une phase grasse comprenant au moins une huile choisie parmi les huiles hydrocarbonées d'origine végétale, de préférence les triglycérides et au moins une huile choisie parmi les alcanes linéaires en C7-C17 ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de poudres d'acide aminé N-acylé ayant un groupe acyle en C8-C22, de préférence avec un rapport massique R silice / acide aminé N-acylé ayant un groupe acyle en C8-C22 supérieur ou égal à 0,75, et encore plus préférentiellement compris entre 0,75 et 3 ; et
- de 40 % à 85 % en poids d'au moins une phase grasse comprenant de 25 % à 48 % en poids d'au moins une huile choisie parmi les huiles hydrocarbonées d'origine végétale, de préférence les triglycérides et de 5 % à 25 % en poids, de préférence de 5 % à 10 % en poids d'au moins une huile choisie parmi les alcanes linéaires en C7-C17 ;
les quantités en poids étant données par rapport au poids total de la composition.

Selon un deuxième mode de réalisation spécifique de l'invention, la composition anhydre comprend :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de les particules sphériques de cellulose, de préférence avec un rapport massique R silice / cellulose étant supérieur ou égal à 0,75, et encore plus préférentiellement compris entre 0,75 et 3 ; et
- de 40 % à 85 % en poids d'au moins une phase grasse comprenant au moins une huile choisie parmi les huiles hydrocarbonées d'origine végétale, de préférence les triglycérides, les alcools de Guerbet, les esters d'acide gras en C8-C30 et d'alcool de Guerbet, et les alcanes linéaires en C7-C17 ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de les particules sphériques de cellulose, de préférence avec un rapport massique R silice / cellulose supérieur ou égal à 0,75, et encore plus préférentiellement compris entre 0,75 et 3 ; et
- de 40 % à 85 % en poids d'au moins une phase grasse comprenant au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de les particules sphériques de cellulose, de préférence avec un rapport massique R silice / cellulose supérieur ou égal à 0,75, et encore plus préférentiellement compris entre 0,75 et 3 ; et
- de 40 % à 85 % en poids d'au moins une phase grasse comprenant de 25 % à 70 % en poids, de préférence de 25 % à 48 % en poids, d'au moins une huile choisie parmi les alcools de Guerbet et les esters d'acide gras en C8-C30 et d'alcool de Guerbet ;
les quantités en poids étant données par rapport au poids total de la composition.

Selon un troisième mode de réalisation spécifique de l'invention, la composition anhydre comprend :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 10 % à 50 % en poids de charges dont au moins 5 % en poids de microparticules sphériques de silice poreuse et au moins 5 % en poids de les particules sphériques de cellulose, de préférence avec un rapport massique R silice / cellulose supérieur ou égal à 0,75, et encore plus préférentiellement compris entre 0,75 et 3 ; et
- de 40 % à 85 % en poids d'au moins une phase grasse comprenant au moins une huile choisie parmi les huiles hydrocarbonées d'origine végétale, de préférence les triglycérides, les alcools de Guerbet et les esters d'acide gras en C8-C30 et d'alcool de Guerbet, et les alcanes linéaires en C7-C17 ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 10 % à 50 % en poids de charges dont au moins 5 % en poids de microparticules sphériques de silice poreuse et au moins 5 % en poids de les particules sphériques de cellulose, de préférence avec un rapport massique R silice / cellulose supérieur ou égal à 0,75, et encore plus préférentiellement compris entre 0,75 et 3 ; et
- de 40 % à 85 % en poids d'au moins une phase grasse comprenant au moins une huile choisie parmi les huiles hydrocarbonées d'origine végétale, de préférence les triglycérides et au moins une huile choisie parmi les alcanes linéaires en C7-C17 ;
les quantités en poids étant données par rapport au poids total de la composition.

De préférence, la composition anhydre comprend :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 10 % à 50 % en poids de charges dont au moins 5 % en poids de microparticules sphériques de silice poreuse et au moins 5 % en poids de les particules sphériques de cellulose, de préférence avec un rapport massique R silice / cellulose étant supérieur ou égal à 0,75, et encore plus préférentiellement compris entre 0,75 et 3 ; et
- de 40 % à 85 % en poids d'au moins une phase grasse comprenant de 25 % à 48 % en poids d'au moins une huile choisie parmi les huiles hydrocarbonées d'origine végétale, de préférence les triglycérides et de 5 % à 25 % en poids, de préférence de 5 % à 10 % en poids d'au moins une huile choisie parmi les alcanes linéaires en C7-C17 ;
les quantités en poids étant données par rapport au poids total de la composition.

Selon un quatrième mode de réalisation spécifique de l'invention, la composition anhydre comprend :
- de 3 % à 15 % en poids d'au moins un gélifiant lipophile choisi parmi les esters de dextrine et d'acide gras, de préférence le myristate de dextrine ;
- de 10 % à 50 % en poids de charges dont au moins 5 % en poids de poudres d'acide aminé N-acylé ayant un groupe acyle en C8-C22 et au moins 5 % en poids de particules sphériques de cellulose ; et
- de 40 % à 85 % en poids d'au moins une phase grasse comprenant au moins une huile choisie parmi les huiles hydrocarbonées d'origine végétale, de préférence les triglycérides, les alcools de Guerbet et les esters d'acide gras en C8-C30 et d'alcool de Guerbet, et les alcanes linéaires en C7-C17 ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le myristate de dextrine ;
- de 10 % à 50 % en poids de charges dont au moins 5 % en poids de poudres d'acide aminé N-acylé ayant un groupe acyle en C8-C22 et au moins 5 % en poids de particules sphériques de cellulose ; et
- de 40 % à 85 % en poids d'au moins une phase grasse comprenant au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le myristate de dextrine ;
- de 10 % à 50 % en poids de charges dont au moins 5 % en poids de poudres d'acide aminé N-acylé ayant un groupe acyle en C8-C22 et au moins 5 % en poids de particules sphériques de cellulose ; et
- de 40 % à 85 % en poids d'au moins une phase grasse comprenant de 5 % à 25 % en poids, de préférence de 5 % à 15 % en poids, d'au moins une huile choisie parmi les alcools de Guerbet et les esters d'acide gras en C8-C30 et d'alcool de Guerbet ;
les quantités en poids étant données par rapport au poids total de la composition.

Selon un cinquième mode de réalisation spécifique de l'invention, la composition anhydre comprend :
- de 3 % à 15 % en poids d'au moins un gélifiant lipophile choisi parmi les esters de dextrine et d'acide gras, de préférence le myristate de dextrine ;
- de 10 % à 50 % en poids de charges dont au moins 5 % en poids de microparticules sphériques de silice poreuse et au moins 5 % en poids de les particules sphériques de cellulose ; et
- de 40 % à 85 % en poids d'au moins une phase grasse comprenant au moins une huile choisie parmi les huiles hydrocarbonées d'origine végétale, de préférence les triglycérides, les alcools de Guerbet et les esters d'acide gras en C8-C30 et d'alcool de Guerbet, et les alcanes linéaires en C7-C17 ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le myristate de dextrine ;
- de 10 % à 50 % en poids de charges dont au moins 5 % en poids de microparticules sphériques de silice poreuse et au moins 5 % en poids de les particules sphériques de cellulose ; et
- de 40 % à 85 % en poids d'au moins une phase grasse comprenant au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le myristate de dextrine ;
- de 10 % à 50 % en poids de charges dont au moins 5 % en poids de microparticules sphériques de silice poreuse et au moins 5 % en poids de les particules sphériques de cellulose ; et
- de 40 % à 85 % en poids d'au moins une phase grasse comprenant de 5 % à 25 % en poids, de préférence de 5 % à 15 % en poids, d'au moins une huile choisie parmi les alcools de Guerbet et les esters d'acide gras en C8-C30 et d'alcool de Guerbet ;
les quantités en poids étant données par rapport au poids total de la composition.

Selon un sixième mode de réalisation spécifique de l'invention, la composition anhydre comprend :
- de 3 % à 15 % en poids d'au moins un gélifiant lipophile choisi parmi les esters de dextrine et d'acide gras, de préférence le myristate de dextrine ;
- de 15 % à 50 % en poids de charges dont au moins trois charges distinctes les unes des autres dont l'une est choisie parmi les charges inorganiques et les deux autres sont choisies parmi les charges organiques, de préférence au moins trois charges distinctes les unes des autres dont l'une est choisie parmi les particules sphériques de silice poreuse, en particulier les microparticules sphériques de silice poreuse et les deux autres sont choisies parmi les particules sphériques de cellulose, les poudres d'acide aminé N-acylé ayant un groupe acyle en C8-C22 et les particules de polyamide, de préférence les particules sphériques de cellulose et les poudres d'acide aminé N-acylé ayant un groupe acyle en C8-C22 ; et
- de 40 % à 85 % en poids d'au moins une phase grasse comprenant au moins une huile choisie parmi les huiles hydrocarbonées d'origine végétale, de préférence les triglycérides, les alcools de Guerbet et les esters d'acide gras en C8-C30 et d'alcool de Guerbet, et les alcanes linéaires en C7-C17 ;
les quantités en poids étant données par rapport au poids total de la composition.

Selon un mode de réalisation particulier, la composition anhydre comprend :
- de 3 % à 15 % en poids d'au moins un gélifiant lipophile choisi parmi les esters de dextrine et d'acide gras, de préférence le myristate de dextrine ;
- de 15 % à 50 % en poids de charges dont au moins trois charges distinctes les unes des autres dont l'une est choisie parmi les charges inorganiques et les deux autres sont choisies parmi les charges organiques, de préférence au moins trois charges distinctes les unes des autres dont l'une est choisie parmi les particules sphériques de silice poreuse, en particulier les microparticules sphériques de silice poreuse et les deux autres sont choisies parmi les particules sphériques de cellulose, les poudres d'acide aminé N-acylé ayant un groupe acyle en C8-C22 et les particules de polyamide, de préférence les particules sphériques de cellulose et les poudres d'acide aminé N-acylé ayant un groupe acyle en C8-C22 ; et
- de 40 % à 85 % en poids d'au moins une phase grasse comprenant au moins une huile choisie parmi les alcools de Guerbet et les esters d'acide gras en C8-C30 et d'alcool de Guerbet ;
les quantités en poids étant données par rapport au poids total de la composition.

De préférence, la composition anhydre comprend :
- de 3 % à 15 % en poids d'au moins un gélifiant lipophile choisi parmi les esters de dextrine et d'acide gras, de préférence le myristate de dextrine ;
- de 15 % à 50 % en poids de charges dont au moins trois charges distinctes les unes des autres dont l'une est choisie parmi les charges inorganiques et les deux autres sont choisies parmi les charges organiques, de préférence au moins trois charges distinctes les unes des autres dont l'une est choisie parmi les particules sphériques de silice poreuse, en particulier les microparticules sphériques de silice poreuse et les deux autres sont choisies parmi les particules sphériques de cellulose, les poudres d'acide aminé N-acylé ayant un groupe acyle en C8-C22 et les particules de polyamide, de préférence les particules sphériques de cellulose et les poudres d'acide aminé N-acylé ayant un groupe acyle en C8-C22 ; et
- de 40 % à 85 % en poids d'au moins une phase grasse comprenant de 5 % à 25 % en poids, de préférence de 5 % à 15 % en poids, et encore plus préférentiellement de 5 % à 10 % en poids, d'au moins une huile choisie parmi les alcools de Guerbet et les esters d'acide gras en C8-C30 et d'alcool de Guerbet ;
les quantités en poids étant données par rapport au poids total de la composition.

Encore plus préférentiellement, la composition anhydre comprend :
- de 3 % à 15 % en poids d'au moins un gélifiant lipophile choisi parmi les esters de dextrine et d'acide gras, de préférence le myristate de dextrine ;
- de 15 % à 50 % en poids d'au moins trois charges distinctes les unes des autres dont au moins 5 % en poids d'une première charge inorganique choisie parmi les particules sphériques de silice poreuse, en particulier les microparticules sphériques de silice poreuse au moins 5 % en poids d'au moins une deuxième charge organique choisie parmi les particules sphériques de cellulose, et au moins 5 % en poids d'une troisième charge organique choisie parmi les poudres d'acide aminé N-acylé ayant un groupe acyle en C8-C22 ; et
- de 40 % à 85 % en poids d'au moins une phase grasse comprenant de 5 % à 25 % en poids, de préférence de 5 % à 15 % en poids, et encore plus préférentiellement de 5 % à 10 % en poids, d'au moins une huile choisie parmi les alcools de Guerbet et les esters d'acide gras en C8-C30 et d'alcool de Guerbet ;
les quantités en poids étant données par rapport au poids total de la composition.

La composition selon l'invention peut en outre contenir un ou plusieurs polyols comprenant de 2 à 8 atomes de carbone. Par « polyols », il faut comprendre toute molécule organique comportant au moins deux groupements hydroxyle libres. Comme polyols, on peut citer par exemple la glycérine, les glycols comme le butylène glycol, le propylène glycol, l'isoprène glycol, le dipropylène glycol, l'hexylène glycol, les polyéthylènes glycols, le polypropylène glycol et notamment le dipropylène glycol, le propane-1,2-diol, le propane-1,3-diol. Selon un mode de réalisation particulier de l'invention, le polyol est choisi parmi la glycérine et le propane-1,3-diol. De préférence, le polyol est la glycérine.

On peut citer à titre d'exemple la glycerine commercialisée sous la dénomination GLYCERINE 4810 par la société OLEON ou encore sous la dénomination PALMERA G995V par la société KLK OLEO ou encore sous la dénomination REFINED GLYCERINE 99,5% PH. EURO par la société CARGILL.
On peut également citer le propane-1,3-diol commercialisé sous la dénomination ZEMEA PROPANEDIOL par la société Dupont Tate and Lyle Bio Products
On peut encore citer le butylène glycol commercialisé sous la dénomination 1,3 BUTYLENE GLYCOL par la société ALZO ou encore DAICEL.

On peut aussi citer le propylène glycol commercialisé sous la dénomination RADIANOL 4710 par la société OLEON

La quantité en polyols peut aller par exemple de 0 % à 20 % en poids, de préférence de 3 % à 17 % en poids, mieux de 4 % à 15 % en poids, et encore mieux de 5 % à 10 % en poids par rapport au poids total de la composition.

La composition conforme à l'invention peut également comprendre un ou plusieurs alcools primaires, c'est-à-dire un alcool comportant de 1 à 6 atomes de carbone, tel que l'éthanol, le propanol, l'isopropanol, le butanol, le pentanol, l'hexanol et en particulier l'éthanol et l'isopropanol. Il s'agit de préférence de l'éthanol. L'ajout d'un tel alcool peut être notamment approprié lorsque la composition selon l'invention est utilisée comme produit pour le corps, le visage, ou les cheveux.

La quantité en alcools primaires peut aller par exemple de 0 % à 35 % en poids, de préférence de 1 à 15 % en poids, et encore plus préférentiellement de 5 à 10 % en poids par rapport au poids total de la composition.

La composition de l'invention peut contenir un ou plusieurs des adjuvants habituels dans les domaines cosmétique et dermatologique tels que des tensioactifs ; des agents hydratants ; des actifs hydrophiles ou lipophiles ; des agents anti-radicaux libres ; des antioxydants ; des conservateurs ; des parfums ; des agents filmogènes ; des nacres ; des pigments ; des colorants ; et leurs mélanges.

La composition conforme à l'invention peut comprendre un ou plusieurs actifs.

On peut citer à titre d'exemple d'actif, et de façon non limitative, l'acide ascorbique et ses dérivés tels que le 5,6-di-O-diméthylsilylascorbate (notamment vendu par la Société Exsymol sous la référence PRO-AA), le sel de potassium du dl-alpha-tocopheryl-2l-ascorbyl-phosphate (notamment vendu par la Société Senju Pharmaceutical sous la référence SEPIVITAL EPC), l'ascorbyl phosphate de magnésium, l'ascorbyl phosphate de sodium (notamment vendu par la Société Roche sous la référence Stay-C 50) ; le phloroglucinol ; les enzymes ; et leurs mélanges. Parmi les actifs hydrophiles sensibles à l'oxydation, on utilise selon un mode de réalisation préféré de l'invention l'acide ascorbique. L'acide ascorbique peut être de toute nature. Ainsi, il peut être d'origine naturelle sous forme de poudre ou sous forme de jus d'orange de préférence concentré. Il peut être aussi d'origine synthétique, de préférence sous forme de poudre.

Comme autres actifs utilisables dans la composition de l'invention, on peut citer par exemple les agents hydratants tels que les hydrolysats de protéines ; les extraits naturels ; les anti-inflammatoires ; les oligomères procyannidoliques ; les vitamines comme la vitamine A (rétinol), la vitamine E (tocophérol) et autres anti-oxydants tel l'extrait de romarin, la vitamine B5 (panthénol), la vitamine B3 (niacinamide), les dérivés de ces vitamines (notamment esters) et leurs mélanges ; l'urée ; la caféine ; l'adenosine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens ; les agents matifiants comme les fibres ; les agents tenseurs, les cires ; les filtres UV ; les huiles essentielles ; les céramides ; et leurs mélanges.

Les quantités de ces différents adjuvants et/ou actifs sont celles classiquement utilisées dans les domaines considérés. En particulier, ces quantités varient selon le but recherché et peuvent par exemple aller de 0,01 % à 20 %, et de préférence de 0,1 % à 10 % en poids du poids total de la composition.

Bien entendu, l'homme de l'art veillera à choisir le ou les éventuels adjuvants et/ou actifs ajoutés à la composition selon l'invention de manière telle que les propriétés avantageuses attachées intrinsèquement à la composition conforme à l'invention ne soient pas, ou substantiellement pas, altérées par l'addition envisagée.

La composition selon l'invention peut être utilisée pour toute application topique ; notamment, elle peut constituer une composition cosmétique ou dermatologique, de préférence une composition cosmétique, et en particulier dans le domaine cosmétique à efficacité perçue immédiate. Elle peut en particulier être utilisée pour le soin et/ou le démaquillage de la peau, des lèvres et /ou des yeux, et également comme composition pour le soin des cheveux. Elle peut également être utilisée comme déodorant ou comme produit solaire, ainsi que pour le nettoyage de la peau.

La composition conforme à l'invention peut notamment être utilisée comme produit à usage cosmétique pour le soin de la peau sur les axes anti-âge, peaux grasses, protection solaire, antiperspirants et déodorants, les produits cheveux et/ou cuir chevelu ainsi que styling, les produits parfumants ainsi que des produits de maquillage.

L'invention a aussi pour objet l'utilisation cosmétique d'une composition telle que définie ci-dessus, pour le soin, le démaquillage et/ou le nettoyage de la peau, des lèvres et/ou des yeux, et/ou pour le soin des cheveux.

La présente invention a encore pour objet un procédé cosmétique de démaquillage et/ou de soin de la peau, des lèvres et/ou des yeux, dans lequel on applique sur la peau, les lèvres et/ou les yeux, une composition telle que définie ci-dessus.

La présente invention a aussi pour objet un procédé cosmétique de soin des cheveux, dans lequel on applique sur les cheveux, une composition telle que définie ci-dessus.

La présente invention a également pour objet un procédé de traitement cosmétique de camouflage des imperfections colorielles et/ou des imperfections de relief de la peau.

Selon un mode préféré de réalisation de l'invention, la composition constitue une composition pour le soin de la peau du corps et / ou du visage, de préférence du visage.

La présente invention a également pour objet une composition aqueuse sous la forme d'une dispersion d'au moins une composition anhydre telle que définie précédemment dans une phase aqueuse.

La composition conforme à l'invention peut être obtenue de manière classique par l'homme du métier.

Selon un mode de réalisation particulier, la composition conforme à l'invention est obtenue selon le procédé suivant :
1) Mouillage à froid du gélifiant par la phase grasse ;
2) Mise en chauffe jusqu'à obtenir une température de 70 °C à 80 °C suivant le cas ;
3) Gélification à 70 °C à 80 °C suivant le cas pendant 15 minutes sous émulseur, vitesse maximum adaptée au volume fabriqué ;
4) Refroidissement jusqu'à obtenir une température de 35 °C ;
5) Ajout des charges à 35 °C, sous émulseur. Dispersion jusqu'à disparition des amas et obtention d'un jus homogène, lisse et mat d'aspect macroscopique ;
6) Vidange de cuve.

Selon un autre mode de réalisation particulier, la composition conforme à l'invention est obtenue selon le procédé suivant :
1) Mise en chauffe de la phase grasse jusqu'à obtenir une température de 70 °C à 80 °C suivant le cas ;
2) Ajout du gélifiant et gélification à 70 °C à 80 °C suivant le cas pendant 15 minutes sous émulseur, vitesse maximum adaptée au volume fabriqué ;
3) Refroidissement jusqu'à obtenir une température de 35 °C ;
4) Ajout des charges à 35 °C. Dispersion jusqu'à disparition des amas et obtention d'un jus homogène, lisse et mat d'aspect macroscopique.
5) Vidange de cuve.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières, sauf mention contraire.

### Exemples

Les compositions 1 à 12 suivantes ont été réalisées.

| **Composition** | **1** | **2** | **3** | **4** | **5** |
|---|---|---|---|---|---|
| SILICA (Silica Beads SB-300 de la société MYOSHI KASEI) | 25 | 15 | 17,5 | 25 | - |
| LAUROYL LYSINE (AMIHOPE LL de AJINOMOTO) | 10 | 20 | - | - | 7 |
| CELLULOSE (CELLULOBEADS USF de DAITO KASEI) | - | - | 12,5 | 10 | 8 |
| DEXTRIN PALMITATE (RHEOPEARL TL2-OR de CHIBA FLOUR MILLING) | 10 | 10 | 8,5 | - | - |
| DEXTRIN MYRISTATE (RHEOPEARL MKL2 de CHIBA FLOUR MILLING) | - | - | - | - | 9,5 |
| TRI HYDROXYSTEARIN (THIXCIN R de ELEMENTIS) | - | - | - | 5 | - |
| CAPRYLIC/CAPRIC TRIGLYCERIDE (MYRITOL 318 de COGNIS (BASF) | 26,95 | 30,95 | - | 46 | - |
| OCTYLDODECYL MYRISTATE (MOD de GATTEFOSSE) | - | - | 37 | - | - |
| ISOPROPYL MYRISTATE (Isopropyl Myristate de COGNIS) | - | - | - | - | 30,1 |
| OCTYLDODECANOL (EUTANOL G de COGNIS) | - | - | - | - | 10 |
| PRUNUS ARMENIACA KERNEL OIL (LIPOVOL P de LIPO CHEMICALS) | 10 | 10 | 10 | - | 10 |
| ISOPROPYL PALMITATE (Isopropyl palmitate de COGNIS) | 13 | 14 | 14,45 | - | - |
| COCO-CAPRYLATE/CAPRATE (CETIOL LC de COGNIS) | - | - | - | - | 10 |
| DICAPRYLYL ETHER (CETIOL OE de COGNIS) | - | - | - | - | 10 |
| UNDECANE (and) TRIDECANE (CETIOL UT de COGNIS) | 5 | - | - | 9 | - |
| CANDELILLA CERA (CANDELILLA WAX SP 75 G de STRAHL & PITSCH) | - | - | - | 5 | - |
| ALCOHOL | - | - | - | - | 5 |
| PARFUM | - | - | - | - | 0,3 |
| Antioxydant | 0,05 | 0,05 | 0,05 | - | 0,1 |

| **Composition** | **6** | **7** | **8** | **9** | **10** |
|---|---|---|---|---|---|
| SILICA (Silica Beads SB-300 de la société MYOSHI KASEI) | 5 | 5 | 5 | 5 | - |
| LAUROYL LYSINE (AMIHOPE LL de AJINOMOTO) | - | 5 | 5 | 5 | 7 |
| CELLULOSE (CELLULOBEADS USF de DAITO KASEI) | 10 | 5 | 5 | 5 | 8 |
| DEXTRIN PALMITATE (RHEOPEARL TL2-OR de CHIBA FLOUR MILLING) | - | - | - | - | - |
| DEXTRIN MYRISTATE (RHEOPEARL MKL2 de CHIBA FLOUR MILLING) | 8,5 | 8,5 | 8,5 | 8,5 | 8,5 |
| ISOPROPYL MYRISTATE (Isopropyl Myristate de COGNIS) | 52 | 24,1 | 19,1 | 19,1 | 24,1 |
| OCTYLDODECANOL (EUTANOL G de COGNIS) | 14,1 | 10 | 10 | 10 | 10 |
| PRUNUS ARMENIACA KERNEL OIL (LIPOVOL P de LIPO CHEMICALS) | 10 | 10 | 10 | 5 | 10 |
| HYDROGENATED COCO-GLYCERIDES (SOFTISAN 100 de CREMER OLEO) | - | 1 | 5 | 5 | 1 |
| COCO-CAPRYLATE/CAPRATE (CETIOL LC de COGNIS) | - | 10 | 10 | 10 | 10 |
| GLYCERYL DIBEHENATE (and) TRIBEHENIN (and) GLYCERYL BEHENATE (COMPRITOL 888 CG ATO de GATTEFOSSE) | - | 2 | 3 | 3 | 2 |
| DICAPRYLYL ETHER (CETIOL OE de COGNIS) | - | 10 | 10 | 10 | 10 |
| GLYCERIN | - | - | - | 5 | - |
| GLYCERYLSTEARATE (DUB GMS 50/50 de Stearinerie Dubois) | - | 4 | 4 | 4 | 4 |
| ALCOHOL | - | 5 | 5 | 5 | 5 |
| PARFUM | 0,3 | 0,3 | 0,3 | 0,3 | 0,3 |
| Anti-oxydant | 0,1 | 0,1 | 0,1 | 0,1 | 0,1 |

| **Composition** | **11** | **12** | | | |
|---|---|---|---|---|---|
| SILICA (Silica Beads SB-300 de la société MYOSHI KASEI) | 15 | 15 | | | |
| LAUROYL LYSINE (AMIHOPE LL de AJINOMOTO) | 20 | 20 | | | |
| Colorant(s) | qs | qs | | | |
| DEXTRIN PALMITATE (RHEOPEARL TL2-OR de CHIBA FLOUR MILLING) | 10 | 10 | | | |
| CAPRYLIC/CAPRIC TRIGLYCERIDE (MYRITOL 318 de COGNIS) | 27,7 | 26,7 | | | |
| PRUNUS ARMENIACA KERNEL OIL (LIPOVOL P de LIPO CHEMICALS) | 7 | 7 | | | |
| ISOPROPYL PALMITATE (Isopropyl palmitate de COGNIS) | 14 | 14 | | | |
| CERA ALBA (CERABEIL LOR de BAERLOCHER) | - | 1 | | | |
| GLYCERIN | 3 | 3 | | | |
| ALCOHOL | 3 | 3 | | | |
| Antioxydant | 0,3 | 0,3 | | | |

### Procédé de préparation

La phase grasse est homogénéisée. Après introduction du gélifiant, phase de gélification une fois la température souhaitée atteinte (70 °C à 80 °C selon le cas).

Phase de refroidissement puis ajout de tout autre ingrédient de la formule le cas échéant puis des charges (température allant de 50 °C à 32 °C).

### Caractérisation des propriétés optiques

Les propriétés optiques des compositions 1 à 10 ont été caractérisées à l'aide de la mesure de Flou ou de Haze (effet voile ou calque) avec un appareil commercial de type « Hazemeter ».

Les mesures ont été effectuées selon le protocole suivant : sur un film transparent plastique (Byk), une couche de 25,4 µm d'épaisseur humide de la composition dont on cherche à évaluer le Flou est étalée, à l'aide d'un étaleur automatique. On laisse sécher pendant 1 heure à température ambiante puis on procède à la mesure de l'indice de flou (ou Haze) à l'aide d'un HAZE GARD de marque BYK GARDNER.

Les valeurs obtenues pour les compositions 1 à 10 sont les suivantes :

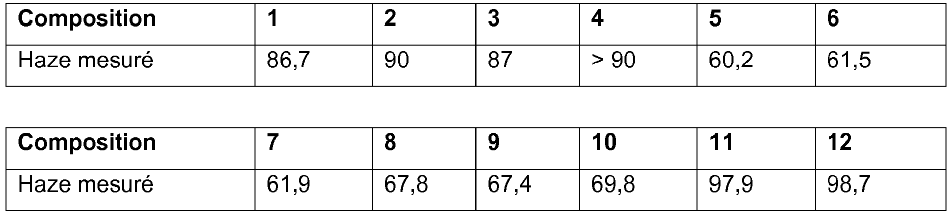

A titre indicatif, il est considéré qu'entre 20 et 60, on a obtenu un faible effet soft-focus, entre 60 et 80, on a obtenu un bon effet soft focus, et à partir de 90, on a obtenu un très bon effet soft-focus.

Ces mesures montrent donc que les compositions conformes à l'invention permettent d'obtenir un effet soft focus important.

### Evaluation sensorielle :

Les propriétés sensorielles des compositions 1 à 12 ont été évaluées après leur application sur la peau. Les résultats sont résumés dans le tableau suivant :

| Composition | Propriétés sensorielles |
|---|---|
| 1 | pénétration rapide, non collant, non brillant, sans film ni film blanc, fini peau velouté |
| 2 | pénétration rapide, non brillant, non collant, sans film, fini peau très doux et sensation de poudré. |
| 3 | pénétration rapide, non collant, non brillant, fini peau poudré. |
| 4 | Non évaluée |
| 5 | pénétration assez lente, fini peau doux, très légèrement collant, effet filmogène au toucher huileux. |
| 6 | fini peau poudré et doux, sec, pas de collant. |
| 7 | pénétration lente, texture grasse à transformation en toucher doux, très légèrement filmogène et collant. |
| 8 | pénétration assez rapide, non brillante, non collante, non filmogène, fini peau poudré. |
| 9 | pénétration assez lente au glissant modéré, légèrement filmogène et collante, fini peau poudré. |
| 10 | pénétration rapide, légèrement brillante, non collante, assez filmogène, fini peau poudré. |
| 11 | pénétration rapide, non brillant, non collant, fini peau très doux et sensation de poudré. |
| 12 | pénétration rapide, non brillant, non collant, fini peau très doux et sensation de poudré. |

### Stabilité

La stabilité des compositions 1 à 12 a été évaluée sur une période de 2 mois à température ambiante (25 °C).

Toutes les compositions sont stables après 2 mois de stockage à 25 °C.

## Revendications

1. Composition anhydre comprenant :
- de 3 % à 15 % en poids d'au moins un gélifiant lipophile ;
- de 10 % à 50 % en poids de charges dont au moins 5 % en poids d'une première charge et au moins 5 % en poids d'une deuxième charge distincte de la première ; et
- de 40 % à 85 % en poids d'au moins une phase grasse ;
les quantités en poids étant données par rapport au poids total de la composition,
dans laquelle l'une des charges est choisie parmi les particules sphériques de cellulose, les poudres d'acide aminé N-acylé ayant un groupe acyle en C8-C22, et les particules de polyamide, et l'autre est choisie parmi les particules sphériques de silice poreuse.

2. Composition selon la revendication 1 dans laquelle le ou les gélifiants lipophiles sont choisis parmi les esters de dextrine et d'acide gras et les tri-esters d'acide gras en C8-C30 et de mono ou polyglycérol, de préférence les esters de dextrine et d'acide gras.

3. Composition selon l'une quelconque des revendications 1 à 2 dans laquelle le ou les esters de dextrine et d'acides gras sont choisis parmi les mono- ou poly-esters de dextrine et d'au moins un acide gras répondant à la formule (C) : 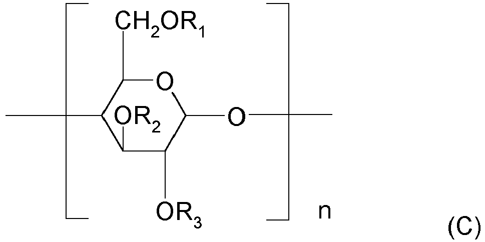 dans laquelle :
n est un entier allant de 3 à 150, notamment de 10 à 100, et de préférence de 15 à 40, les radicaux R₁, R₂ et R₃, représentent un atome d'hydrogène ou un groupement acyle (R-CO-) dans lequel le radical R est un groupement hydrocarboné, linéaire ou ramifié, saturé ou insaturé, possédant 6 à 50, notamment 8 à 30, voire 12 à 22 et mieux 12 à 18 atomes de carbone, sous réserve qu'au moins un desdits radicaux R₁, R₂ ou R₃ est différent d'un atome d'hydrogène.

4. Composition selon la revendication 3 dans laquelle le ou les esters de dextrine et d'acides gras est un composé de formule (C) dans laquelle :
n varie avantageusement de 25 à 35, de préférence va de 27 à 33, et mieux est égal à 30, le radical R-CO- est choisi parmi les radicaux caprylyle, caproyle, lauroyle, myristyle, palmityle, stéaryle, eicosanyle, docosanoyle, isovaléryle, éthyl-2 butyryle, éthylméthylacétyle, isoheptanyle, éthyl-2 hexanyle, isononanyle, isodécanyle, isotridécanyle, isomyristyle, isopalmityle, isostéaryle, isohexanyle, décènyle, dodécenyle, tétradécényle, myristyle, hexadécénoyle, palmitolyle, oléyle, élaidyle, eicosényle, sorbyle, linoléyle, linolényle, punicyle, arachidonyle, stéarolyle, et leurs mélanges, de préférence le radical R-CO- est le radical palmityle ou le radical myristyle, et encore plus préférentiellement le radical palmityle.

5. Composition selon l'une quelconque des revendications 1 à 4 dans laquelle l'acide gras du ou des tri-esters d'acide gras en C8-C30 et de mono ou polyglycérol est un acide linéaire ou ramifié, saturé ou insaturé, comprenant de 10 à 24 atomes de carbone, préférentiellement de 12 à 22, et mieux de 16 à 20 atomes de carbone, substitué ou non substitué par un ou plusieurs groupements hydroxyle, de préférence un acide linéaire, saturé, substitué par au moins un groupement hydroxyle.

6. Composition selon l'une quelconque des revendications 1 à 5 dans laquelle le ou les tri-esters d'acide gras en C8-C30 et de mono ou polyglycérol sont choisis parmi les tri-esters d'acide gras en C8-C30 et de monoglycérol, de préférence les tri(hydroxystéarate) de glycéryle, et encore plus préférentiellement le tris(12-hydroxystéarate) de glycéryle.

7. Composition selon l'une quelconque des revendications 1 à 6 dans laquelle la poudre d'acide aminé N-acylé ayant un groupe acyle en C8-C22 est la lauroyl lysine.

8. Composition selon l'une quelconque des revendications 1 à 7 dans laquelle les particules sphériques de silice poreuse sont des microparticules sphériques de silice poreuse.

9. Composition selon l'une quelconque des revendications 1 à 8 comprenant au moins deux charges distintes l'une de l'autre dont l'une est choisie parmi les particules sphériques de cellulose et l'autre est choisie parmi les particules sphériques de silice poreuse, en particulier les microparticules sphériques de silice poreuse.

10. Composition selon l'une quelconque des revendications 1 à 8 comprenant au moins deux charges distintes l'une de l'autre dont l'une est choisie parmi les poudres d'acide aminé N-acylé ayant un groupe acyle en C8-C22 et l'autre est choisie parmi les particules sphériques de silice poreuse, en particulier les microparticules sphériques de silice poreuse.

11. Composition selon l'une quelconque des revendications 1 à 8 comprenant au moins trois charges distinctes l'une de l'autre dont l'une est choisie parmi les particules sphériques de silice poreuse, en particulier les microparticules sphériques de silice poreuse et les deux autres sont choisies parmi les particules sphériques de cellulose, les poudres d'acide aminé N-acylé ayant un groupe acyle en C8-C22 et les particules de polyamide, de préférence les particules sphériques de cellulose et les poudres d'acide aminé N-acylé ayant un groupe acyle en C8-C22.

12. Composition selon l'une quelconque des revendications 1 à 11 comprenant au moins une charge choisie parmi les particules sphériques de silice poreuse, en particulier les microparticules sphériques de silice poreuse, le rapport massique R silice / charges distinctes de la silice étant supérieur ou égal à 0,75, de préférence compris entre 0,75 et 3.

13. Composition selon l'une quelconque des revendications 1 à 12 dans laquelle la phase grasse comprend au moins une huile, de préférence choisie parmi les huiles hydrocarbonées d'origine végétale telles que les triglycérides, les alcools de Guerbet et les esters d'acide gras en C8-C30 et d'alcool de Guerbet, et les alcanes linéaires en C7-C17.

14. Composition selon la revendication 13 comprenant de 15 % à 85 % en poids d'au moins une huile choisie parmi les huiles hydrocarbonées d'origine végétale, de préférence les triglycérides, les alcools de Guerbet et les esters d'acide gras en C8-C30 et d'alcool de Guerbet, et éventuellement de 5 % à 25 % en poids, de préférence de 5 % à 10 % en poids d'au moins une huile choisie parmi les alcanes linéaires en C7-C17.

15. Composition anhydre selon l'une quelconque des revendications 1 à 14 comprenant :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de poudres d'acide aminé N-acylé ayant un groupe acyle en C8-C22, le rapport massique R silice / acide aminé N-acylé ayant un groupe acyle en C8-C22 étant supérieur ou égal à 0,75, de préférence compris entre 0,75 et 3 ; et
- de 40 % à 85 % en poids d'au moins une phase grasse comprenant de 25 % à 48 % en poids d'au moins une huile choisie parmi les huiles hydrocarbonées d'origine végétale, de préférence les triglycérides ;
les quantités en poids étant données par rapport au poids total de la composition.

16. Composition anhydre selon l'une quelconque des revendications 1 à 14 comprenant :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de poudres d'acide aminé N-acylé ayant un groupe acyle en C8-C22, le rapport massique R silice / acide aminé N-acylé ayant un groupe acyle en C8-C22 étant supérieur ou égal à 0,75, de préférence compris entre 0,75 et 3 ; et
- de 40 % à 85 % en poids d'au moins une phase grasse comprenant de 25 % à 48 % en poids d'au moins une huile choisie parmi les huiles hydrocarbonées d'origine végétale, de préférence les triglycérides et de 5 % à 25 % en poids, de préférence de 5 % à 10 % en poids d'au moins une huile choisie parmi les alcanes linéaires en C7-C17 ;
les quantités en poids étant données par rapport au poids total de la composition.

17. Composition anhydre selon l'une quelconque des revendications 1 à 14 comprenant :
- de 3 % à 15 % en poids d'au moins un gélifiant lipophile choisi parmi les esters de dextrine et d'acide gras, de préférence le palmitate de dextrine ;
- de 10 % à 50 % en poids de charges dont au moins 5 % en poids de microparticules sphériques de silice poreuse et au moins 5 % en poids de les particules sphériques de cellulose, le rapport massique R silice / cellulose étant supérieur ou égal à 0,75, de préférence compris entre 0,75 et 3 ; et
- de 40 % à 85 % en poids d'au moins une phase grasse comprenant de 25 % à 70 % en poids, de préférence de 25 % à 48 % en poids d'au moins une huile choisie parmi les alcools de Guerbet et les esters d'acide gras en C8-C30 et d'alcool de Guerbet ;
les quantités en poids étant données par rapport au poids total de la composition.

18. Composition anhydre selon l'une quelconque des revendications 1 à 14 comprenant :
- de 3 % à 15 % en poids d'au moins un gélifiant lipophile choisi parmi les tri-esters d'acide gras en C8-C30 et de mono ou polyglycérol tels que le tri-hydroxy-stéarate de glycéryle ;
- de 10 % à 50 % en poids de charges dont au moins 5 % en poids de microparticules sphériques de silice poreuse et au moins 5 % en poids de les particules sphériques de cellulose, le rapport massique R silice / cellulose étant supérieur ou égal à 0,75, de préférence compris entre 0,75 et 3 ; et
- de 40 % à 85 % en poids d'au moins une phase grasse comprenant de 25 % à 48 % en poids d'au moins une huile choisie parmi les huiles hydrocarbonées d'origine végétale, de préférence les triglycérides et de 5 % à 25 % en poids, de préférence de 5 % à 10 % en poids d'au moins une huile choisie parmi les alcanes linéaires en C7-C17 ;
les quantités en poids étant données par rapport au poids total de la composition.

19. Procédé de traitement cosmétique des matières kératiniques dans lequel on applique sur les matières kératiniques une composition telle que définie à l'une quelconque des revendications 1 à 18.

20. Composition aqueuse sous la forme d'une dispersion d'au moins une composition anhydre telle que définie à l'une quelconque des revendications 1 à 18 dans une phase aqueuse.

## Patentansprüche

1. Wasserfreie Zusammensetzung, umfassend:
- 3 bis 15 Gew.-% mindestens eines lipophilen Geliermittels;
- 10 bis 50 Gew.-% Füllstoffe, davon mindestens 5 Gew.-% eines ersten Füllstoffs und mindestens 5 Gew.-% eines zweiten Füllstoffs, der von dem ersten Füllstoff verschieden ist; und
- 40 bis 85 Gew.-% mindestens einer Fettphase; wobei sich die Gewichtsmengen auf das Gesamtgewicht der Zusammensetzung beziehen,
wobei einer der Füllstoffe aus kugelförmigen Celluloseteilchen, Pulvern einer N-acetylierten Aminosäure mit einer C8-C22-Acylgruppe und Polyamidteilchen ausgewählt ist und der andere aus kugelförmigen Teilchen von porösem Siliciumdioxid ausgewählt ist.

2. Zusammensetzung nach Anspruch 1, wobei der lipophile Gelbildner bzw. die lipophilen Gelbildner aus Estern von Dextrin und Fettsäure und Triestern von C8-C30-Fettsäure und Mono- oder Polyglycerin, vorzugsweise Estern von Dextrin und Fettsäure, ausgewählt ist bzw. sind.

3. Zusammensetzung nach einem der Ansprüche 1 bis 2, wobei der Ester bzw. die Ester von Dextrin und Fettsäuren aus Mono- oder Polyestern von Dextrin und mindestens einer Fettsäure der Formel (C) ausgewählt ist bzw. sind: 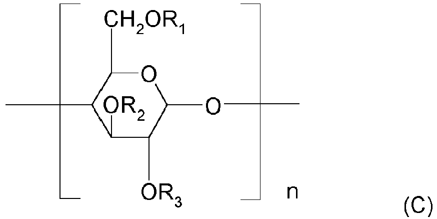 wobei:
n für eine ganze Zahl im Bereich von 3 bis 150, insbesondere von 10 bis 100 und vorzugsweise von 15 bis 40 steht,
die Reste R₁, R₂ und R₃ für ein Wasserstoffatom oder eine Acylgruppe (R-CO-) stehen, in der der Rest R für eine lineare oder verzweigte, gesättigte oder ungesättigte Kohlenwasserstoffgruppe mit 6 bis 50, insbesondere 8 bis 30, sogar 12 bis 22 und noch besser 12 bis 18 Kohlenstoffatomen steht, mit der Maßgabe, dass mindestens einer der Reste R₁, R₂ und R₃ von einem Wasserstoffatom verschieden ist.

4. Zusammensetzung nach Anspruch 3, wobei es sich bei dem Ester bzw. den Estern von Dextrin und Fettsäuren um eine Verbindung der Formel (C) handelt, wobei:
n vorteilhafterweise im Bereich von 25 bis 35 und vorzugsweise im Bereich von 27 bis 33 liegt und noch besser gleich 30 ist,
der Rest R-CO- aus Caprylyl-, Caproyl-, Lauroyl-, Myristyl-, Palmityl-, Stearyl-, Eicosanyl-, Docosanoyl-, Isovaleryl-, 2-Ethylbutyryl-, Ethylmethylacetyl-, Isoheptanyl-, 2-Ethylhexanyl-, Isononanyl-, Isodecanyl-, Isotridecanyl-, Isomyristyl-, Isopalmityl-, Isostearyl-, Isohexanyl-, Decenyl-, Dodecenyl-, Tetradecenyl-, Myristyl-, Hexadecenoyl-, Palmitolyl-, Oleyl-, Elaidyl-, Eicosenyl-, Sorbyl-, Linoleyl-, Linolenyl-, Punicyl-, Arachidonyl- und Steroylresten und Mischungen davon ausgewählt ist, wobei es sich bei dem Rest R-CO- vorzugsweise um den Palmitylrest oder den Myristylrest und noch weiter bevorzugt um den Palmitylrest handelt.

5. Zusammensetzung nach einem der Ansprüche 1 bis 4, wobei es sich bei der Fettsäure des Triesters bzw. der Triester von C8-C30-Fettsäure und Mono- oder Polyglycerin um eine lineare oder verzweigte, gesättigte oder ungesättigte Säure mit 10 bis 24 Kohlenstoffatomen, vorzugsweise 12 bis 22 und noch besser 16 bis 20 Kohlenstoffatomen, die gegebenenfalls durch eine oder mehrere Hydroxylgruppen substituiert ist, vorzugsweise um eine lineare, gesättigte Säure, die durch mindestens eine Hydroxylgruppe substituiert ist, handelt.

6. Zusammensetzung nach einem der Ansprüche 1 bis 5, wobei der Triester bzw. die Triester von C8-C30-Fettsäure und Mono- oder Polyglycerin aus Triestern von C8-C30-Fettsäure und Monoglycerin, vorzugsweise Glyceryltri(hydroxystearat) und noch weiter bevorzugt Glyceryltris(12-hydroxystearat) ausgewählt ist bzw. sind.

7. Zusammensetzung nach einem der Ansprüche 1 bis 6, wobei es sich bei dem Pulver einer N-acetylierten Aminosäure mit einer C8-C22-Acylgruppe um Lauroyllysin handelt.

8. Zusammensetzung nach einem der Ansprüche 1 bis 7, wobei es sich bei den kugelförmigen Teilchen von porösem Siliciumdioxid um kugelförmige Mikroteilchen von porösem Siliciumdioxid handelt.

9. Zusammensetzung nach einem der Ansprüche 1 bis 8, umfassend mindestens zwei voneinander verschiedene Füllstoffe, von denen einer aus kugelförmigen Celluloseteilchen ausgewählt ist und der andere aus kugelförmigen Teilchen von porösem Siliciumdioxid, insbesondere kugelförmigen Mikroteilchen aus porösem Siliciumdioxid, ausgewählt ist.

10. Zusammensetzung nach einem der Ansprüche 1 bis 8, umfassend mindestens zwei voneinander verschiedene Füllstoffe, von denen einer aus Pulvern einer N-acetylierten Aminosäure mit einer C8-C22-Acylgruppe ausgewählt ist und der andere aus kugelförmigen Teilchen von porösem Siliciumdioxid, insbesondere kugelförmigen Mikroteilchen aus porösem Siliciumdioxid, ausgewählt ist.

11. Zusammensetzung nach einem der Ansprüche 1 bis 8, umfassend mindestens drei voneinander verschiedene Füllstoffe, von denen einer aus kugelförmigen Teilchen von porösem Siliciumdioxid, insbesondere kugelförmigen Mikroteilchen aus porösem Siliciumdioxid, ausgewählt ist und die beiden anderen aus kugelförmigen Celluloseteilchen, Pulvern einer N-acetylierten Aminosäure mit einer C8-C22-Acylgruppe und Polyamidteilchen, vorzugsweise kugelförmigen Celluloseteilchen und Pulvern einer N-acetylierten Aminosäure mit einer C8-C22-Acylgruppe, ausgewählt ist.

12. Zusammensetzung nach einem der Ansprüche 1 bis 11, umfassend mindestens einen Füllstoff, der aus kugelförmigen Teilchen von porösem Siliciumdioxid, insbesondere kugelförmigen Mikroteilchen von porösem Siliciumdioxid, ausgewählt ist, wobei das Gewichtsverhältnis R von Siliciumdioxid zu Füllstoffen, die von Siliciumdioxid verschieden sind, größer oder gleich 0,75 ist und vorzugsweise zwischen 0,75 und 3 liegt.

13. Zusammensetzung nach einem der Ansprüche 1 bis 12, wobei die Fettphase mindestens ein Öl umfasst, das vorzugsweise aus Kohlenwasserstoffölen pflanzlicher Herkunft wie Triglyceriden, Guerbet-Alkoholen und Estern von C8-C30-Fettsäure und Guerbet-Alkohol und linearen C7-C17-Alkanen ausgewählt ist.

14. Zusammensetzung nach Anspruch 13, umfassend 15 bis 85 Gew.-% mindestens eines Öls, das aus Kohlenwasserstoffölen pflanzlicher Herkunft, vorzugsweise Triglyceriden, Guerbet-Alkoholen und Estern von C8-C30-Fettsäure und Guerbet-Alkohol, ausgewählt ist, und gegebenenfalls 5 bis 25 Gew.-%, vorzugsweise 5 bis 10 Gew.-%, mindestens eines Öls, das aus linearen C7-C17-Alkanen ausgewählt ist.

15. Wasserfreie Zusammensetzung nach einem der Ansprüche 1 bis 14, umfassend:
- 3 bis 15 Gew.-% mindestens eines lipophilen Geliermittels, das aus Estern von Dextrin und Fettsäure, vorzugsweise Dextrinpalmitat, ausgewählt ist;
- 10 bis 50 Gew.-% Füllstoffe, davon mindestens 5 Gew.-% kugelförmige Mikroteilchen von porösem Siliciumdioxid und mindestens 5 Gew.-% Pulvern einer N-acetylierten Aminosäure mit einer C8-C22-Acylgruppe, wobei das Gewichtsverhältnis R von Siliciumdioxid zu N-acetylierter Aminosäure größer oder gleich 0,75 ist und vorzugsweise zwischen 0,75 und 3 liegt; und
- 40 bis 85 Gew.-% mindestens einer Fettphase, umfassend 25 bis 48 Gew.-% mindestens eines Öls, das aus Kohlenwasserstoffölen pflanzlicher Herkunft, vorzugsweise Triglyceriden, ausgewählt ist;
wobei sich die Gewichtsmengen auf das Gesamtgewicht der Zusammensetzung beziehen.

16. Wasserfreie Zusammensetzung nach einem der Ansprüche 1 bis 14, umfassend:
- 3 bis 15 Gew.-% mindestens eines lipophilen Geliermittels, das aus Estern von Dextrin und Fettsäure, vorzugsweise Dextrinpalmitat, ausgewählt ist;
- 10 bis 50 Gew.-% Füllstoffe, davon mindestens 5 Gew.-% kugelförmige Mikroteilchen von porösem Siliciumdioxid und mindestens 5 Gew.-% Pulvern einer N-acetylierten Aminosäure mit einer C8-C22-Acylgruppe, wobei das Gewichtsverhältnis R von Siliciumdioxid zu N-acetylierter Aminosäure mit einer C8-C22-Acylgruppe größer oder gleich 0,75 ist und vorzugsweise zwischen 0,75 und 3 liegt; und
- 40 bis 85 Gew.-% mindestens einer Fettphase, umfassend 25 bis 48 Gew.-% mindestens eines Öls, das aus Kohlenwasserstoffölen pflanzlicher Herkunft, vorzugsweise Triglyceriden, ausgewählt ist, und 5 bis 25 Gew.-%, vorzugsweise 5 bis 10 Gew.-%, eines Öls, das aus linearen C7-C17-Alkanen ausgewählt ist;
wobei sich die Gewichtsmengen auf das Gesamtgewicht der Zusammensetzung beziehen.

17. Wasserfreie Zusammensetzung nach einem der Ansprüche 1 bis 14, umfassend:
- 3 bis 15 Gew.-% mindestens eines lipophilen Geliermittels, das aus Estern von Dextrin und Fettsäure, vorzugsweise Dextrinpalmitat, ausgewählt ist;
- 10 bis 50 Gew.-% Füllstoffe, davon mindestens 5 Gew.-% kugelförmige Mikroteilchen von porösem Siliciumdioxid und mindestens 5 Gew.-% kugelförmige Celluloseteilchen, wobei das Gewichtsverhältnis R von Siliciumdioxid zu N-acetylierter Aminosäure größer oder gleich 0,75 ist und vorzugsweise zwischen 0,75 und 3 liegt; und
- 40 bis 85 Gew.-% mindestens einer Fettphase, umfassend 25 bis 70 Gew.-%, vorzugsweise 25 bis 48 Gew.-%, mindestens eines Öls, das aus Guerbet-Alkoholen und Estern von C8-C30-Fettsäure und Guerbet-Alkohol ausgewählt ist;
wobei sich die Gewichtsmengen auf das Gesamtgewicht der Zusammensetzung beziehen.

18. Wasserfreie Zusammensetzung nach einem der Ansprüche 1 bis 14, umfassend:
- 3 bis 15 Gew.-% mindestens eines lipophilen Geliermittels, das aus Triestern von C8-C30-Fettsäure und Mono- oder Polyglycerinen wie Glyceryltrihydroxystearat ausgewählt ist;
- 10 bis 50 Gew.-% Füllstoffe, davon mindestens 5 Gew.-% kugelförmige Mikroteilchen von porösem Siliciumdioxid und mindestens 5 Gew.-% kugelförmige Celluloseteilchen, wobei das Gewichtsverhältnis R von Siliciumdioxid zu N-acetylierter Aminosäure größer oder gleich 0,75 ist und vorzugsweise zwischen 0,75 und 3 liegt; und
- 40 bis 85 Gew.-% mindestens einer Fettphase, umfassend 25 bis 48 Gew.-% mindestens eines Öls, das aus Kohlenwasserstoffölen pflanzlicher Herkunft, vorzugsweise Triglyceriden, ausgewählt ist, und 5 bis 25 Gew.-%, vorzugsweise 5 bis 10 Gew.-%, mindestens eines Öls, das aus linearen C7-C17-Alkanen ausgewählt ist;
wobei sich die Gewichtsmengen auf das Gesamtgewicht der Zusammensetzung beziehen.

19. Verfahren zur kosmetischen Behandlung von Keratinmaterialien, bei dem man auf die Keratinmaterialien eine Zusammensetzung gemäß einem der Ansprüche 1 bis 18 aufbringt.

20. Wässrige Zusammensetzung in Form einer Dispersion mindestens einer wasserfreien Zusammensetzung gemäß einem der Ansprüche 1 bis 18 in einer wässrigen Phase.

## Claims

1. Anhydrous composition comprising:
- from 3% to 15% by weight of at least one lipophilic gelling agent;
- from 10% to 50% by weight of fillers, including at least 5% by weight of a first filler and at least 5% by weight of a second filler different from the first; and
- from 40% to 85% by weight of at least one fatty phase; the amounts by weight being given relative to the total weight of the composition,
in which one of the fillers is chosen from spherical cellulose particles, powders of an N-acylated amino acid having a C₈-C₂₂ acyl group, and polyamide particles, and the other is chosen from spherical particles of porous silica.

2. Composition according to Claim 1, in which the lipophilic gelling agent(s) are chosen from esters of dextrin and of a fatty acid and triesters of a C₈-C₃₀ fatty acid and of mono- or polyglycerol, preferably esters of dextrin and of a fatty acid.

3. Composition according to either one of Claims 1 and 2, in which the ester (s) of dextrin and of fatty acids are chosen from mono- or polyesters of dextrin and of at least one fatty acid corresponding to formula (C): 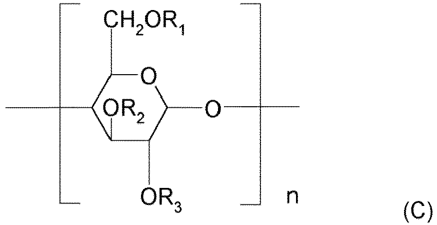 in which:
n is an integer ranging from 3 to 150, in particular from 10 to 100, and preferably from 15 to 40, and the R₁, R₂ and R₃ radicals represent a hydrogen atom or an acyl group (R-CO-) in which the R radical is a linear or branched,
saturated or unsaturated hydrocarbon-based group containing 6 to 50, in particular 8 to 30, or even 12 to 22 and better still 12 to 18 carbon atoms, with the proviso that at least one of said R₁, R₂ or R₃ radicals is other than a hydrogen atom.

4. Composition according to Claim 3, in which the ester(s) of dextrin and of fatty acids is a compound of formula (C) in which:
n advantageously ranges from 25 to 35, preferably ranges from 27 to 33, and better still is equal to 30, and the R-CO- radical is chosen from caprylyl, caproyl, lauroyl, myristyl, palmityl, stearyl, eicosanyl, docosanoyl, isovaleryl, 2-ethylbutyryl, ethylmethylacetyl, isoheptanyl, 2-ethylhexanyl, isononanyl, isodecanyl, isotridecanyl, isomyristyl, isopalmityl, isostearyl, isohexanyl, decenyl, dodecenyl, tetradecenyl, myristyl, hexadecenoyl, palmitolyl, oleyl, eladiyl, eisenyl, sorbyl, linoleyl, linolenyl, punicyl, arachidonyl and stearoyl radicals, and mixtures thereof, the R-CO-radical preferably being the palmityl radical or the myristyl radical, and even more preferentially the palmityl radical.

5. Composition according to any one of Claims 1 to 4, in which the fatty acid of the triester(s) of a C₈-C₃₀ fatty acid and of mono- or polyglycerol is a linear or branched, saturated or unsaturated acid comprising from 10 to 24 carbon atoms, preferentially from 12 to 22, and better still from 16 to 20 carbon atoms, which is unsubstituted or substituted with one or more hydroxyl groups, preferably a linear, saturated acid substituted with at least one hydroxyl group.

6. Composition according to any one of Claims 1 to 5, in which the triester(s) of C₈-C₃₀ fatty acid and of mono- or polyglycerol are chosen from triesters of a C₈-C₃₀ fatty acid and of monoglycerol, preferably glyceryl tri(hydroxystearates), and even more preferentially glyceryl tris(12-hydroxystearate).

7. Composition according to any one of Claims 1 to 6, in which the powder of an N-acylated amino acid having a C₈-C₂₂ acyl group is lauroyl lysine.

8. Composition according to any one of Claims 1 to 7, in which the spherical particles of porous silica are spherical microparticles of porous silica.

9. Composition according to any one of Claims 1 to 8, comprising at least two fillers different from one another, at least one of which is chosen from spherical cellulose particles and the other is chosen from spherical particles of porous silica, in particular spherical microparticles of porous silica.

10. Composition according to any one of Claims 1 to 8, comprising at least two fillers different from one another, at least one of which is chosen from powders of an N-acylated amino acid having a C₈-C₂₂ acyl group and the other is chosen from spherical particles of porous silica, in particular spherical microparticles of porous silica.

11. Composition according to any one of Claims 1 to 8, comprising at least three fillers different from one another, at least one of which is chosen from spherical particles of porous silica, in particular spherical microparticles of porous silica, and the other two are chosen from spherical cellulose particles, powders of an N-acylated amino acid having a C₈-C₂₂ acyl group and polyamide particles, preferably spherical cellulose particles and powders of an N-acylated amino acid having a C₈-C₂₂ acyl group.

12. Composition according to any one of Claims 1 to 11, comprising at least one filler chosen from spherical particles of porous silica, in particular spherical microparticles of porous silica, the weight ratio R of silica/fillers different from silica being greater than or equal to 0.75, preferably between 0.75 and 3.

13. Composition according to any one of Claims 1 to 12, in which the fatty phase comprises at least one oil, preferably chosen from hydrocarbon-based oils of plant origin, such as triglycerides, Guerbet alcohols and esters of a C₈-C₃₀ fatty acid and of a Guerbet alcohol, and linear C₇-C₁₇ alkanes.

14. Composition according to Claim 13, comprising from 15% to 85% by weight of at least one oil chosen from hydrocarbon-based oils of plant origin, preferably triglycerides, Guerbet alcohols and esters of a C₈-C₃₀ fatty acid and of a Guerbet alcohol, and optionally from 5% to 25% by weight, preferably from 5% to 10% by weight of at least one oil chosen from linear C₇-C₁₇ alkanes.

15. Anhydrous composition according to any one of Claims 1 to 14, comprising:
- from 3% to 15% by weight of at least one lipophilic gelling agent chosen from esters of dextrin and of a fatty acid, preferably dextrin palmitate;
- from 10% to 50% by weight of fillers, including at least 5% by weight of spherical microparticles of porous silica and at least 5% by weight of powders of an N-acylated amino acid having a C₈-C₂₂ acyl group, the weight ratio R of silica/N-acylated amino acid having a C₈-C₂₂ acyl group being greater than or equal to 0.75, preferably between 0.75 and 3; and
- from 40% to 85% by weight of at least one fatty phase comprising from 25% to 48% by weight of at least one oil chosen from hydrocarbon-based oils of plant origin, preferably triglycerides;
the amounts by weight being given relative to the total weight of the composition.

16. Anhydrous composition according to any one of Claims 1 to 14, comprising:
- from 3% to 15% by weight of at least one lipophilic gelling agent chosen from esters of dextrin and of a fatty acid, preferably dextrin palmitate;
- from 10% to 50% by weight of fillers, including at least 5% by weight of spherical microparticles of porous silica and at least 5% by weight of powders of an N-acylated amino acid having a C₈-C₂₂ acyl group, the weight ratio R of silica/N-acylated amino acid having a C₈-C₂₂ acyl group being greater than or equal to 0.75, preferably between 0.75 and 3; and
- from 40% to 85% by weight of at least one fatty phase comprising from 25% to 48% by weight of at least one oil chosen from hydrocarbon-based oils of plant origin, preferably triglycerides, and from 5% to 25% by weight, preferably from 5% to 10% by weight of at least one oil chosen from linear C₇-C₁₇ alkanes;
the amounts by weight being given relative to the total weight of the composition.

17. Anhydrous composition according to any one of Claims 1 to 14, comprising:
- from 3% to 15% by weight of at least one lipophilic gelling agent chosen from esters of dextrin and of a fatty acid, preferably dextrin palmitate;
- from 10% to 50% by weight of fillers, including at least 5% by weight of spherical microparticles of porous silica and at least 5% by weight of spherical cellulose particles, the silica/cellulose weight ratio R being greater than or equal to 0.75, preferably between 0.75 and 3; and
- from 40% to 85% by weight of at least one fatty phase comprising from 25% to 70% by weight, preferably from 25% to 48% by weight of at least one oil chosen from Guerbet alcohols and esters of a C₈-C₃₀ fatty acid and of a Guerbet alcohol;
the amounts by weight being given relative to the total weight of the composition.

18. Anhydrous composition according to any one of Claims 1 to 14, comprising:
- from 3% to 15% by weight of at least one lipophilic gelling agent chosen from triesters of a C₈-C₃₀ fatty acid and of mono- or polyglycerol, such as glyceryl trihydroxystearate;
- from 10% to 50% by weight of fillers, including at least 5% by weight of spherical microparticles of porous silica and at least 5% by weight of spherical cellulose particles, the silica/cellulose weight ratio R being greater than or equal to 0.75, preferably between 0.75 and 3; and
- from 40% to 85% by weight of at least one fatty phase comprising from 25% to 48% by weight of at least one oil chosen from hydrocarbon-based oils of plant origin, preferably triglycerides, and from 5% to 25% by weight, preferably from 5% to 10% by weight of at least one oil chosen from linear C₇-C₁₇ alkanes;
the amounts by weight being given relative to the total weight of the composition.

19. Method for the cosmetic treatment of keratin materials, in which a composition as defined in any one of Claims 1 to 18 is applied to the keratin materials.

20. Aqueous composition in the form of a dispersion of at least one anhydrous composition as defined in any one of Claims 1 to 18 in an aqueous phase.
